(19) Europäisches Patentamt
European Patent Office
Office européen des brevets

(11) Publication number : **0 167 245**
**B1**

(12) # EUROPEAN PATENT SPECIFICATION

(45) Date of publication of patent specification : **16.06.87**

(51) Int. Cl.⁴ : **C 07 C143/78**, C 07 C143/76, C 07 C143/822, A 61 K 31/16

(21) Application number : **85303309.0**

(22) Date of filing : **10.05.85**

(54) **Anti-arrhythmic agents.**

(30) Priority : **11.05.84 US 609151**
**29.03.85 US 717341**
**29.03.85 US 717739**

(43) Date of publication of application :
**08.01.86 Bulletin 86/02**

(45) Publication of the grant of the patent :
**16.06.87 Bulletin 87/25**

(84) Designated contracting states :
**AT BE CH DE FR IT LI LU NL SE**

(56) References cited :
**FR-A- 2 374 300**

(73) Proprietor : **AMERICAN HOME PRODUCTS CORPORATION**
**685, Third Avenue**
**New York, New York 10017 (US)**

(72) Inventor : **Buzby, George Carroll**
**997 Stoneybrook Drive, Blue Bell**
**Montgomery, PA (US)**

(74) Representative : **Connelly, Michael John et al**
**John Wyeth & Brother Limited Huntercombe Lane**
**South Taplow**
**Maidenhead Berkshire, SL6 0PH (GB)**

**EP 0 167 245 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

The invention relates to novel compounds useful as pharmaceuticals, particularly as anti-arrhythmic agents, and pharmaceutical compositions containing them.

Pharmacological agents possessing the ability to block cellular transmembrane influx of calcium are capable of suppressing that portion of myocardial or vascular smooth muscle contractility which is dependent upon extracellular calcium. Church et al., Can. J. Physiol. Pharmacol., 58, 254 (1980) ; Fleckenstein, Calcium and the Heart, P. Harris and L. Opie, eds., Academic Press (1971) ; Nayler et al., Bas. Res. Cardiol., 76, 1 (1981) ; Calcium Blockers, S. Flaim and R. Zelis, eds., Urban and Schwartzenberg, (1982).

These pharmacological agents, termed calcium entry blockers, have been proven to be useful in the treatment of hypertension, cardiac arrhythmias, angina pectoris, and coronary artery vasospasm (a possible cause of sudden cardiac death syndrome). Circ. Res., 52, Suppl. I, (1983) ; Hypertension 5, Suppl. II, (1983). In theory, calcium entry blockers are thought to act by blocking calcium influx through discrete calcium channels (slow channels) in cell membranes. Various tissues exhibit relative differences in sensitivity toward the calcium blocking effect achieved by certain calcium antagonists, theoretically as a result of tissue specific differences in the calcium channels. Acta Pharmacol. Toxicol. 43, 5 (1978) ; loc. cit. 291 (1978) ; Microvascular Res., 5, 73 (1973) ; Am. Rev. Pharmacol. Toxicol., 17, 149 (1977). It is believed that the slow calcium current is responsible for activation of pacemaker cells in the sinoatrial node and the atrioventricular node of the heart. Verapamil, a known calcium channel blocking agent, is believed to slow conduction velocity through the atrioventricular node of the heart, in explanation of the mechanism of its antiarrhythmic activity.

In accordance with this invention there is provided a group of new compounds of the formula :

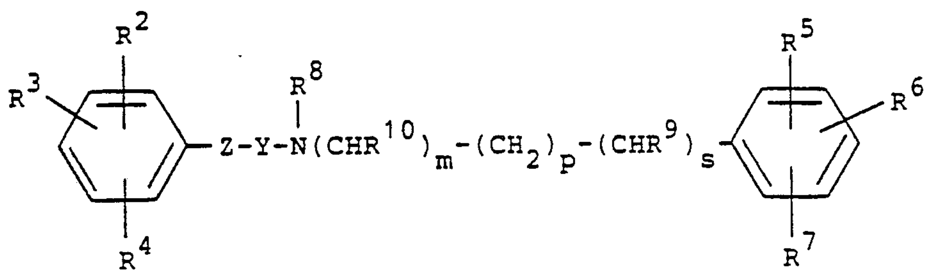

(I)

in which

Y is $-(CH_2)_n-$ where n is 1, 2, 3 or 4 or

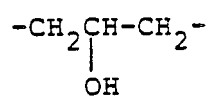

(preferably $-(CH_2)_n-$ where n is 2, 3 or 4) ;

Z is

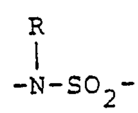

or

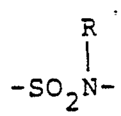

where R is hydrogen, alkyl of 1-6 carbon atoms, polyfluorinated alkyl of 1 to 6 carbon atoms, —CN, cyanoalkyl in which the alkyl moiety contains 1 to 3 carbon atoms, cycloalkyl of 3 to 6 carbon atoms or cycloalkylalkyl of 4 to 8 carbon atoms (preferably alkyl of 1 to 4 carbon atoms or cycloalkyl of 3 to 6 carbon atoms) ;

$R^2$, $R^3$, $R^4$, $R^5$, $R^6$ and $R^7$, independently, are hydrogen, alkoxy of 1 to 3 carbon atoms, trifluoromethyl, —Cl, —Br or —F ;

$R^8$ is hydrogen or alkyl of 1 to 6 carbon atoms (preferably alkyl of 1 to 3 carbon atoms) ;

$R^9$ is alkyl of 1 to 3 carbon atoms ;

$R^{10}$ is alkyl of 1 to 3 carbon atoms ;

m is 0 or 1 (preferably 0) ;
p is 0, 1, 2 or 3 (preferably 1, 2 or 3) ; and
s is 0 or 1 (preferably 0) ;

or a pharmaceutically acceptable salt thereof, with the provisos that the sum of m, p and s must be 1, 2, 3 or 4 ; and the number of linear atoms in the bridge connecting the two aromatic rings does not exceed 9 ; and when Y is methylene or dimethylene, two of $R^2$, $R^3$ and $R^4$ are other than hydrogen.

The preferred compounds of the invention from the standpoint of production economics and activity profile may be grouped as those in which (1) the aromatic ring to the left of the depicted structure contains three substituents in the 2-, 3- and 4-positions or the 3-, 4- and 5-positions where, preferably those substituents are lower alkoxy (most preferably methoxy), Y is trimethylene, m and s are 0 and p is 2 ; (2) the aromatic ring to the left of the depicted structure contains two substituents, either in the 3, 5- or 3, 4-positions, where preferably those substituents are lower alkoxy (most preferably methoxy) or halo (most preferably —Cl), Y is di, tri- or tetra- methylene, m and s are 0 and p is 2 ; and (3) the aromatic ring to the left of the depicted structure contains one substituent, in meta position, Y is trimethylene, m and s are 0 and p is 2.

As subgeneric aspects of the invention there may be mentioned the compounds having formula I and their pharmaceutically acceptable salts where either (i) the number of linear atoms in the bridge connecting the two aromatic rings does not exceed 8 or (ii) Y is tetramethylene whilst m and s are 0 and p is 2. As preferred subgeneric aspects of the invention there may be mentioned compounds having the formula Ia, Ib, Ic and Id

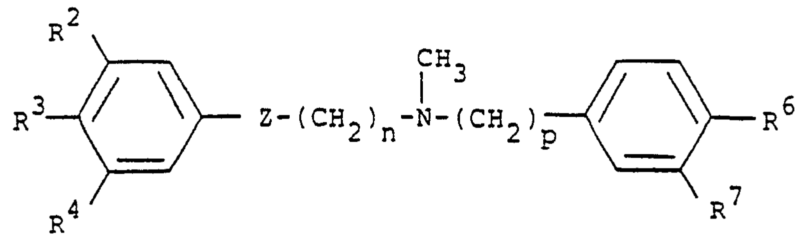

(Ia)

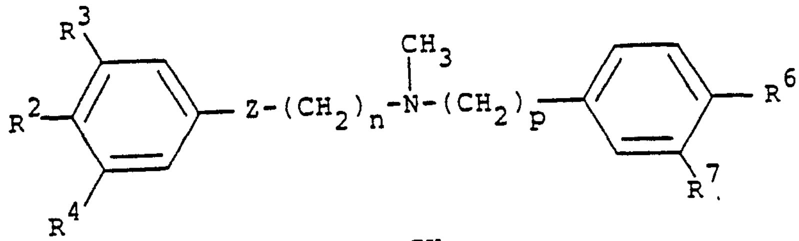

(Ib)

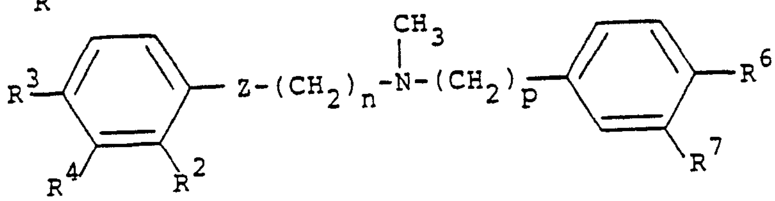

(Ic)

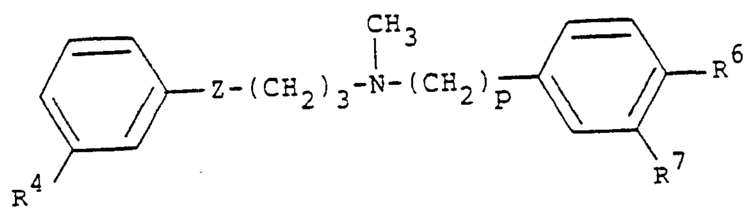

(Id)

in which

Z represents

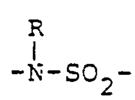

or

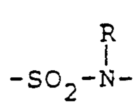

where R is alkyl of 1 to 4 carbon atoms ;

$R^2$ is hydrogen or alkoxy of 1 to 3 carbon atoms ;

$R^3$, $R^4$, $R^6$ and $R^7$ are, independently, alkoxy of 1 to 3 carbon atoms, trifluoromethyl, —Cl, —Br or —F ;

n is 2, 3 or 4 and p is 1 or 2 ;

and the pharmaceutically acceptable salts thereof. As aspects of these preferred compounds there may be mentioned the compounds of formulae Ia and Ib (where n is 2 or 3) and of formula Id and their pharmaceutically acceptable salts.

The pharmaceutically acceptable salts of the antiarrhythmic agents of this invention are prepared directly by neutralization of the free base or by metathetical displacement. The physiologically acceptable salts may be formed with organic or inorganic acids such as hydrochloric, hydrobromic, phosphoric, sulphuric, sulphonic, nitric, methylsulphonic, acetic, maleic, succinic, fumaric, tartaric, citric, salicylic, lactic, naphthalenesulphonic acid, and the like.

The new compounds having formula I contain a secondary or tertiary amine function and an N-substituted thioamide function. The compounds may therefore be prepared by various methods available for the preparation of secondary and tertiary amines (alkylation of amines, reductive amination of carbonyl compounds and reduction of Schiff's bases, amides and thioamides) or for the preparation of N-substituted sulphonamides (sulphonylation of amines and alkylation of sulphonamides).

In the following description to explain the methods that may be used to prepare the compounds of the invention the following symbols will be used for brevity, namely Ar to represent the group having formula XIX

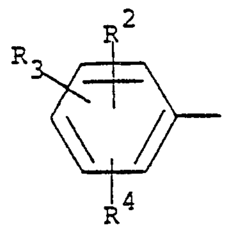

(XIX)

(where $R^{2\text{-}4}$ are defined supra) ; $R^{11}$ to represent any meaning of R other than hydrogen ; $R^{12}$ represent hydrogen or alkyl of 1 to 5 carbon atoms ; $A^1$ to represent hydrogen or alkyl of 1 to 3 carbon atoms ; $A^2$ to represent such a group that $A^1A^2CH$— is the same as the substituted lower alkyl group having the formula

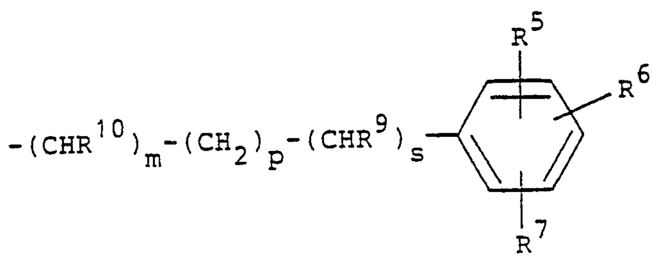

(XX)

(where $R^5$, $R^6$, $R^7$, $R^9$, $R^{10}$, m, p and s are as defined supra) ; X to represent O or S ; $Y^1$ to represent the same group as Y save that if a hydroxy group is present it may be protected by a removable protecting group and q to represent the integer n — 1 where n is 2, 3 or 4, i. e. q is 1, 2 or 3. All other symbols such as Z, $R^8$, and R have the same meaning as in formula I.

The invention provides a process for the preparation of a compound having formula I or a pharmaceutically acceptable salt thereof, which comprises

(1) sulphonylation of an aromatic amine Ar-NHR to introduce the sulphonyl group having the formula II ;

$$—SO_2—Y^1—NR^8—CHA^1A^2 \quad \text{(II)}$$

or

(2) sulphonylation of an amine having the formula III

$$H\overset{*}{N}R—Y^1—NR^8—CHA^1A^2 \quad \text{(III)}$$

to introduce a sulphonyl group having the formula $Ar—SO_2—$ at the nitrogen atom marked with an

asterisk ; or

(3) alkylation of a sulphonamide having the formula

$$Ar—SO_2—NHR \qquad (IV)$$

to introduce a substituted alkyl group having the formula V ;

$$—Y^1—NR^8—CHA^1A^2 \qquad (V)$$

or

(4) alkylation of an amine having the formula VI

$$Ar—Z—Y^1—\overset{*}{N}HR^8 \qquad (VI)$$

to introduce a substituted alkyl group having the formula $—CHA^1A^2$ at the nitrogen atom marked with an asterisk ; or

(5) reductive amination of a carbonyl compound having the formula

$$A^1A^2C{=}O \qquad (VII)$$

with an amine having the formula VI as illustrated above ; or

(6) reduction of a Schiff's base having the formula VIII or IX

$$Ar—Z—Y^1—N{=}CA^1A^2 \qquad (VIII)$$

$$Ar—Z—(CH_2)_q—C{=}N—CHA^1A^2 \qquad (IX)$$

or of an amide or thioamide having formula X or XI ;

$$Ar—Z—Y^1—NR^8—CX—A^2 \qquad (X)$$

$$Ar—Z—(CH_2)_q—CX—NR^8—CHA^1A^2 \qquad (XI)$$

or

(7) alkylation of an amine having the formula XII

$$HNR^8—CHA^1A^2 \qquad (XII)$$

to introduce a substituted alkyl group having the formula XIII ;

$$Ar—Z—Y^1— \qquad (XIII)$$

or

(8) reductive amination of an aldehyde having the formula XIV

$$Ar—Z—(CH_2)_q—CHO \qquad (XIV)$$

with an amine having the formula XII as illustrated above ; or

(9) alkylation of a sulphonamide having the formula XV or XVI

$$Ar—SO_2—\overset{*}{N}H—Y^1—NR^8—CHA^1A^2 \qquad (XV)$$

$$Ar—\overset{*}{N}H—SO_2—Y^1—NR^8—CHA^1A^2 \qquad (XVI)$$

to introduce an alkyl or substituted alkyl group $R^{11}$ at the nitrogen atom marked with an asterisk ; or

(10) reductive amination of an alkanone containing 1 to 6 carbon atoms with an amine having the formula XVII ;

$$Ar—Z—Y^1—NH—CHA^1A^2 \qquad (XVII)$$

or

(11) reduction of an amide or thioamide having the formula XVIII

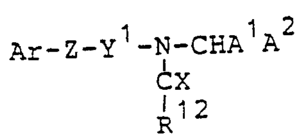

(XVIII)

and, where appropriate, removal of a removable protecting group. The process may also include removal of the acid from an acid addition salt to form the free base, metathetical displacement to convert one salt into another or neutralisation of the free base to form a salt.

In order to prepare the compounds of the invention a sulphonylation reaction will normally be carried out at some stage of the synthesis. Where Y in the final product contains hydroxy, it may be recommended to protect the hydroxy group with a removable protecting group prior to sulphonylation to avoid sulphonylation at the hydroxy group. The hydroxy group is preferably protected by means of formation of its ester derivatives, for instance, acetate ester, which can be hydrolysed at any stage after the sulphonylation to restore the hydroxy group.

The intermediate compounds used in steps (1) to (8) are known compounds or, where new, can be prepared by procedures within the skill of the medicinal chemist. The sulphonamides used in step (9) and the amines used in step (10) are novel compounds provided by this invention and can be prepared by steps (1) to (8). The amides and thioamides used in step (11) can be prepared from the corresponding amines having formula XVI in known manner. The reaction conditions used in the aforesaid process may be those conventionally used for the preparation of N-substituted thioamides or secondary and tertiary amines. In the case of the alkylation reactions the alkylating agent is suitably a compound having the formula P—Q where P is the substituted or unsubstituted alkyl group to be introduced and Q is a leaving group or atom, preferably halogen, advantageously chlorine or bromine. The alkylation of amines is normally carried out by reacting the amine with the alkylating agent in the presence of a suitable base. The base may be an inorganic base, for instance, an alkali metal carbonate, a tertiary amine, for instance triethylamine, or an excess of the amine reactant. The amine reactant may be used in the form of the amine as such or in the form of a salt such as an alkali metal salt. The alkylation of sulphonamides is generally effected under basic conditions such that the sulphonamide is in salt form. In the case of the sulphonylation reactions the sulphonylating agent is suitably a sulphonyl halide derivative, preferably the sulphonyl chloride of the appropriate sulphonic acid. Reductive amination of carbonyl compounds may be effected with catalytic hydrogen, nascent hydrogen or the Leuckart reaction. Thioamides may be reduced to amines with borane or in a two step procedure comprising alkylation with an alkyl halide followed by reaction with sodium borohydride. Amides may be reduced to amines with lithium aluminium hydride. Schiff's bases may be reduced to amines by catalytic hydrogenation.

The following examples of procedures may be given to illustrate the synthesis of the compounds of the invention. An aromatic amine ArNRH is sulphonylated by reaction with C1—$SO_2$—$Y^1$—C1 and the sulphonylation product Ar—NR—$SO_2$—$Y^1$—C1 is used to alkylate an aralkylamine $A^1A^2CHNR^8H$. This reaction sequence affords the N-arylalkanesulphonamide type of compounds of this series. The benzenesulphonamide type of compounds of this series may be produced by reaction of an appropriately substituted aromatic sulphonamide, for instance, Ar—$SO_2$—NH—$CH(CH_3)_2$, with C1—Y—$NR^8$—$CHA^1A^2$ ; or by reaction of an aromatic sulphonyl halide with an omega-hydroxyalkylamine, optionally followed by N-alkylation, and replacement of the hydroxy group with a halogen and alkylation of the appropriate aralkylamine.

The compounds of this invention exhibit $Ca^{+2}$ antagonism in rabbit aortic smooth muscle when tested by a modified procedure from that described by Brockaert et al., Eur. J. Pharmacol., 53, 281 (1979). Here, transverse strips (10 mm × 2.5 mm) from the thoracic aorta were cut and suspended vertically in a jacketed (37 °C — 50 ml volume) organ bath in physiological saline solution (PSS) aerated with 95 % $O_2$/5 % $CO_2$. The composition of PSS was as follows (mM) : NaCl 112, KCl 5, $NaHCO_3$ 25, $KH_2PO_4$ 1, $MgSO_4$ 1.2, $CaCl_2$ 2.5, dextrose 10. The lower end of each tissue strip was attached to a fixed post and the upper end to a Statham UC-4 transducer. Changes in force development were recorded on a Beckman Dynograph Polygraphic Recorder.

Following equilibration, the muscles were contracted in a depolarizing solution of PSS in which 100 mM KCl was substituted for an equimolar concentration of NaCl. Following attainment of steady-state isometric force (20 min.), the test compound was added to afford a final concentration of $1 \times 10^{-5}$ M. The inhibitory effect, expressed as percent relaxation, was determined from the mean of two experiments twenty minutes after the addition of the compound being tested.

In addition, the compounds of this invention demonstrate an inhibitory influence on arterial $Ca^{+2}$-calmodulin dependent myosin light chain phosphorylation and subsequent contractile protein function when tested in standard experimental procedures for these inhibitory properties.

As such, the compounds of the invention present an activity profile consistent with that of anti-arrhythmic agents, which utility was proven in vivo experiments in the standard experimental animal as follows :

Rats weighing between 400-500 gms were anesthetized with 35-40 mg/kg Na pentobarbital i. p. Rats were close-clipped on the neck and left side prior to cannulation of the jugular vein and tracheotomy. In some experiments, a catheter was introduced into the carotid artery for measurement of arterial blood pressure. Respiration was provided by a Harvard Model 681 respirator at a rate of approximately 55/min and a volume of 4 cc per cycle. The rat was then placed upon its right side and the heart was exposed by making an incision and separating the ribs. 4-0 silk on taper RB-1 needle was passed under the left anterior descending coronary artery (LAD) at a location just under the tip of the left atrial appendage. The suture was left to be tied upon occlusion. Lead II ECG and cardiotachometer output were recorded on a

Beckman R 612.

The rat was allowed to stabilize for several minutes before the administration of drug via the cannulated jugular vein. Compounds were suspended in carbowax, with total dose volumes kept below 0.20-0.25 ml. Fifteen minutes after dosing, the LAD was occluded by tying the suture. This procedure provokes severe ventricular arrhythmias, terminating in ventricular fibrillation and death in approximately 73 percent of animals given vehicle only. Data were analysed based on statistical analysis of heart rate fluctuations. Output from a Beckman cardiotachometer was digitised at 200 msec/pt using a Nicolet 3 091 digital oscilloscope, and the data analysed to yield mean ± variance of the rate for each 1 minute period (300 points). The measured variance for the period 5-11 minutes post-occlusion was well correlated with the severity of the observed ventricular arrhythmias, and provided a quantitive measure for the relative antiarrhythmic effectiveness of the compound being tested.

For the purpose of these coronary ligation (C. L.) experiments, the actual mortality rate, expressed as a percentage of the animals employed, was obtained for purpose of comparison with the mortality rate of 73 percent in vehicle-treated animals.

Thus, these data establish the compounds of this invention as $Ca^{+2}$ antagonists which are useful as antiarrhythmic agents functioning more at the vascular level than other known $Ca^{+2}$ entry blockers. It has been observed that compounds of this invention inhibit arterial $Ca^{+2}$-calmodulin dependent myosin light chain phosphorylation and subsequent contractile protein function.

Based upon the activity profile elicited by the compounds of this invention in the above-described standard scientifically recognised test models, the compounds are established as anti-arrhythmic agents useful in the treatment of cardiac arrhythmias and conditions characterised by coronary arteries vasospasm. For that purpose, the compounds may be administered orally or parenterally in suitable dosage forms compatible with the route of administration, whether oral, intraperitoneal, intramuscular, intravenous, intranasal, buccal, etc. The effective dose range determined in the animal test models has been established at from 1 to about 50 milligrams per kilogram host body weight to be administered in single or plural doses as needed to relieve the arrhythmatic dysfunction. The specific dosage regimen for a given patient will depend upon age, pathological state, severity of dysfunction, size of the patient, etc. Oral administration is performed with either a liquid or solid dosage unit in any conventional form such as tablets, capsules, solutions, etc., which comprise a unit dose (e. g. from about 25 milligrams to about 4 grams) of the active ingredient alone or in combination with adjuvants needed for conventional coating, tableting, solubilising, flavouring or colouring. Parenteral administration with liquid unit dosage forms may be via sterile solutions or suspensions in aqueous or oleagenous medium. Isotonic aqueous vehicle for injection is preferred with or without stabilisers, preservatives and emulsifiers.

Pharmaceutical compositions containing a compound of the invention in association or combination with a pharmaceutically acceptable carrier may be prepared by bringing the compound of the invention into association or combination with the carrier.

The following examples illustrate the preparation of a representative number of compounds of this invention. After each example, the $Ca^{+2}$ antagonist activity of the compound is presented in terms of percent relaxation (P. R.) at $10^{-5}$ M concentration unless indicated otherwise. Similarly, the percentage mortality of standard experimental test animals upon coronary ligation (C. L.) is presented for comparison with the control mortality rate of 73 percent of animals receiving vehicle alone.

## Example 1

N-(3,5-Dimethoxyphenyl)-3-[(2-(3,4-dimethoxyphenyl) ethyl]-methylamino]-N-(1-methylethyl)-1-propanesulphonamide

3,5-Dimethoxyaniline (34.62 g, 0.226 mol) in xylene (500 ml) containing suspended sodium carbonate (12.0 g, 0.113 mol) was treated with 3-chloropropane sulphonyl chloride (40.0 g, 0.226 mol) added dropwise with stirring. Stirring was continued for twenty-four hours and the solvent was stripped to provide a black partially crystalline gum. Filtration through dry-column silica gel combined with crystallisation from ethyl acetate/hexane provided N-(3,5-dimethoxyphenyl)-3-chloro-1-propanesulphonamide (20 g), m. p. 68-71 °C.

N-(3,5-Dimethoxyphenyl)-3-chloro-1-propanesulphonamide prepared in the preceding paragraph (16.56 g, .057 mol) was dissolved in xylene (300 ml) containing potassium carbonate (7.86 g, .057 mol) and N-methylhomoveratrylamine (11.13 g, .057 mol) and the reaction mixture was warmed and stirred three days. Filtration, stripping of solvent and chromatography on dry-column silica gel (1 100 g) with 5 % methanol/ethyl acetate provided crude N-(3,5-dimethoxyphenyl)-3-[(2-(3,4-dimethoxyphenyl)ethyl] methylamino]-1-propanesulphonamide (5.32 g).

The product of the preceding paragraph (2.49 g, .005 5 mol) in dry dimethylformamide (70 ml) was treated with sodium hydride (60 % in mineral oil) (0.22 g, .005 5 mol) and the reaction mixture was stirred for one hour. Isopropyl bromide (0.677 g, .005 5 mol) was added and the solution was stirred overnight. The reaction mixture was stripped and chromatography provided the title product (0.43 g) as a gum containing a trace of methylene chloride.

Analysis for : $C_{25}H_{38}SO_6N_2 \cdot 0.2\ CH_2Cl_2$
Calculated : C, 59.16 ; H, 7.56 ; N, 5.47.
Found : C, 59.26 ; H, 7.59 ; N, 5.39.
P. R. = 90 at $10^{-5}$ M ; 27 at $10^{-6}$ M
C. L. = 50 %

## Example 2

N-(3,4-Dimethoxyphenyl)-3-[[2-(3,4-dimethoxyphenyl) ethyl] methylaminol]-N-(1-methylethyl)-1-propanesulphonamide

The title compound was prepared by the method of the preceding Example with the exception that 3,4-dimethoxyaniline rather than 3,5-dimethoxyaniline was employed as the starting material.
Analysis for : $C_{25}H_{38}SO_6N_2$
Calculated : C, 60.70 ; H, 7.74 ; N, 5.66.
Found : C, 60.39 ; H, 7.96 ; N, 5.67.
P. R. = 74
C. L. = 0 %

## Example 3

3-[[2-(3,4-Dimethoxyphenyl) ethyl] methylamino]-N-(1-methylethyl)-N-(3,4,5-trimethoxyphenyl)-1-propanesulphonamide

3,4,5-Trimethoxyaniline (38.3 g, 0.208 mol) in xylene (800 ml), containing a small amount of methylene chloride to maintain a solution, was treated with sodium carbonate (22.13 g). Then 3-chloropropanesulphonylchloride (36.95 g) was added dropwise with stirring and stirring was continued overnight at room temperature. Cooling, filtration and evaporation provided a semisolid mass which was triturated with diethylether, filtered and the filtrate taken to dryness. Chromatography of the residue obtained from the filtrate was carried out on dry-column silica gel with 50-50 ethyl acetate/hexane. Trituration of the product with diethyl ether provided N-3,4,5-trimethoxyphenyl-3-chloro-1-propanesulphonamide (13.09 g) m. p. 84-85 °C.

N-3,4,5-Trimethoxyphenyl-3-chloro-1-propanesulphonamide (13.06 g, .04 mol) in xylene (300 ml), sodium carbonate (3.18 g, .03 mol), caesium carbonate (3.25 g, .01 mol) and N-methylhomoveratrylamine (7.88 g, .04 mol) were heated together with stirring for four days. Cooling, filtering and stripping provided a crude product (21.60 g) which was triturated with diethyl ether and filtered. Chromatography on alumna with 4 % methanol/ethyl acetate provided 3-[[2-(3,4-dimethoxyphenyl)ethyl]methylamino]-N-(3,4,5-trimethoxyphenyl)-1-propanesulphonamide (6.0 g) as a gum, containing traces of dimethylformamide and ethyl acetate.

3-[[2-(3,4-Dimethoxyphenyl)ethyl]methylamino]-N-(3,4,5-trimethoxyphenyl)-1-propanesulphonamide (6.7 g, .013 9 mol) in dry dimethylformamide (100 ml) was treated with sodium hydride, 60 % in mineral oil, (0.555 g, .013 9 mol) and the reaction mixture was stirred for 1 hour. 2-Bromopropane (1.71 g, .013 9 mol) was added and the solution was warmed overnight. The reaction was then stripped, dissolved in methylene chloride, filtered and evaporated to a gum. Preparative high pressure chromatography provided the title compound (3.40 g).
Analysis for : $C_{26}H_{40}N_2O_7S$
Calculated : C, 59.52 ; H, 7.68 ; N, 5.34.
Found : C, 58.76 ; H, 7.73 ; N, 5.34.
P. R. = 62
C. L. = 8.3 %

A portion of the product of the preceding paragraph was crystallised from diethyl ether to provide a crystalline solid m. p. 65-67 °C.

## Example 4

N-(3,5-Dichlorophenyl)-3-[[2-(3,4-dimethoxyphenyl)ethyl] methylamino]-N-(1-methylethyl)-1-propanesulphonamide

The title compound was prepared from 3,5-dichloro-aniline, following the procedures of the preceding examples.
Analysis for : $C_{23}H_{32}N_2O_4SCl_2$
Calculated : C, 54.87 ; H, 6.41 ; N, 5.56.
Found : C, 55.02 ; H, 6.24 ; N, 5.48.
P. R. = 86
C. L. = 0 %

Example 5

N-(3-Trifluoromethylphenyl)-3-[[2-(3,4-dimethoxyphenyl)-ethyl]methylamino]-1-propanesulphonamide

This compound was prepared from 3-trifluoromethylaniline by the preceding method.
Analysis for : $C_{21}H_{27}N_2O_4SF_3$
Calculated : C, 54.77 ; H, 5.91 ; N, 6.08.
Found : C, 54.57 ; H, 5.58 ; N, 5.85.
P. R. = 51
C. L. = 25 %

Example 6

N-(3-Trifluoromethylphenyl)-3-[[2-(3,4-dimethoxyphenyl) ethyl] methylamino]-N-(1-methylethyl)-1-propanesulphonamide

This material was prepared from the substance described in the preceding example by alkylation as in Example 3 and purified by chromatography. $^1H$ NMR ($CDCl_3$) —1.12 (d, 6, J = 3.5Hz $Me_2$) 2.30 (s, 3, N-Me) 3.84 (d, 6, $(OMe)_2$) 6.72 (m, 3, aryl), 7.56 (m, 4, aryl).
Analysis for : $C_{24}H_{33}N_2O_4SF_3$
Calculated : C, 57.36 ; H, 6.62 ; N, 5.57.
Found : C, 57.16 ; H, 6.67 ; N, 5.51.
P. R. = 78.5
C. L. = 14 %

Example 7

N-[3-[[2-(3,4-Dimethoxyphenyl)ethyl]methylamino]propyl]-N-(1-methylethyl)-3-(trifluoromethyl) benzenesulphonamide

N-isopropyl-3-trifluoromethylbenzenesulphonamide (5.362 g, .02 mol) in dimethylformamide (30 ml) was treated with sodium hydride (60 % in mineral oil), (0.802 g) and stirred for 2 hours. Then N-(3-chloropropyl)-3,4-dimethoxy-N-methylphenethylamine (5.436 g, .02 mol) was added and the reaction mixture was heated overnight. Filtration and removal of solvent provided the crude product (6.95 g). Column chromatography and high pressure liquid chromatography provided the pure product (4.35 g) as a gum. $^1H$ NMR ($CDCl_3$) 1.15 (d, 6, J = 3.5H, $Me_2$) 2.25 (s, 3, N-Me) 3.84 (d, 6, $(OMe)_2$) 6.76 (m, 3, Ar) 7.25 (m, 3, Ar).
Analysis for : $C_{24}H_{33}N_2O_4SF_3$
Calculated : C, 57.36 ; H, 6.62 ; N, 5.57.
Found : C, 57.44 ; H, 6.66 ; N, 5.48.
P. R. = 86.6
C. L. = 33 %

Example 8

N-[3-[[2-(3,4-Dimethoxyphenyl) ethyl] methylamino] propyl]-N-(1-methylethyl)-3-(trifluoromethyl) benzenesulphonamide

N-(3-chloropropyl)-N-(1-methylethyl)-3-trifluoromethylbenzenesulphonamide (23.4 g, .068 mol) and N-methyl-homoveratrylamine (13.29 g, .068 mol) in xylene (250 ml) containing anhydrous potassium carbonate (4.68 g, .034 mol) and caesium carbonate (11.08 g, .034 mol) were heated and stirred for 2 1/2 days. The reaction mixture was cooled, filtered and stripped to provide a gum with a small amount of crystalline material evident. Solution in diethyl ether, filtration of the small amount of solid and concentration of the filtrate provided the crude product (32.91 g). Chromatography first on dry column alumina then silica gel provided the same product as in the preceding example (11.84 g) as a gum.

Example 9

3,4-Dichloro-N-[3-[[2-(3,4-dimethoxyphenyl) ethyl] methylamino] propyl]-N-(1-methylethyl) benzenesulphonamide

N-(3-Chloropropyl)-N-isopropyl-3,4-dichlorobenzene-sulphonamide, prepared by reacting 3,4-dichlorobenzenesulphonyl chloride with 3-hydroxypropylamine followed by N-alkylation with 2-bromopropane and displacement of the hydroxyl group by chlorine, (13.94 g), N-methylhomoveratrylamine (8.23 g), $K_2CO_3$ (2.90 g) and $Cs_2CO_3$ (7.39 g) in xylene (300 ml) was heated and stirred for 5 days. The

reaction mixture was cooled, filtered and stripped to provide a partially solid crude product which was stirred with diethyl ether, filtered and the filtrate stripped to provide 17.11 g. This material was chromatographed on dry-column silica gel (500 g) using ethyl acetate to provide the product which was recrystallised from diethyl ether-hexane to give the impure product (3.95 g) (m. p. 62-64 °C). A second recrystallisation from diethyl ether-hexane provided pure product (3.57 g, m. p. 65-66 °C).

Analysis for : $C_{23}H_{32}N_2Cl_2O_4S$
Calculated : C, 54.86 ; H, 6.41 ; N, 5.56.
Found : C, 54.91 ; H, 6.20 ; N, 5.49.
C. L. = 0 %

## Example 10

N-(3,4-Dichlorophenyl)-3-[[2-(3,4-dimethoxyphenyl) ethyl]-methylamino]-N-(1-methylethyl)-1-propanesulphonamide

Following the procedures of the preceding examples with the exception that 3,4-dichloroaniline was employed as an initial reactant, N-(3,4-dichlorophenyl)-3-[[2-(3,4-dimethoxyphenyl) ethyl] methylamino]-1-propanesulphonamide was obtained. 4.23 g of this compound in dry DMF (40 ml) was treated with 6 % NaH/mineral oil (0.267 g) and stirred for one hour. An appropriate amount of 2-bromopropane was added and the reaction was heated at Variac setting 25 overnight. The reaction was stripped to provide 6.13 g of a crude gum. The crude gum was dissolved in methylene chloride, washed with aqueous sodium bicarbonate, and the organic layer stripped. Chromatography provided the pure product (1.49 g) as a gum.

Analysis for : $C_{23}H_{32}N_2O_4SCl_2$
Calculated : C, 54.87 ; H, 6.41 ; N, 5.56.
Found : C, 54.74 ; H, 6.44 ; N, 5.43.
C. L. = 40 %

## Example 11

N-(3,4,5-Trimethoxyphenyl)-3-[[1-(3,4,5-trimethoxyphenyl)ethyl] amino]-1-propanesulphonamide

3-Amino-N-(3,4,5-trimethoxyphenyl)-1-propanesulphonamide was prepared from 3-chloro-N-(3,4,5-trimethoxyphenyl)-1-propanesulphonamide. This material (3.04 g, .01 mol) and 3,4,5-trimethoxyphenyl acetone (2.10 g, .01 mol) was dissolved in absolute methanol (50 ml) containing platinum oxide 00.40 g) and shaken at 50 psi (3.45 bars) of hydrogen for 16 hours. After filtration and removal of solvent the residue was chromatographed on dry-column silica gel with 5 % methanol-ethyl acetate to provide the product (2.47 g) as a partial ethyl acetate solvate.

Analysis for : $C_{23}H_{34}N_2O_8SO5C_4H_8O_2$
Calculated : C, 55.23 ; H, 7.23 ; N, 5.15.
Found : C, 54.82 ; H, 6.74 ; N, 5.29.
C. L. = 40 %

## Example 12

3-[[(3,4-Dimethoxyphenyl)methyl]methylamino]-N-[3-(trifluoromethyl)phenyl]propanesulphonamide

The title compound was prepared by reaction of 3-chloro-N-3-(trifluoromethyl) phenyl-propanesulphonamide with N-methyl-3,4-dimethoxybenzylamine in xylene as in previous examples.

Analysis for : $C_{20}H_{25}N_2O_4SF_3$
Calculated : C, 53.80 ; H, 5.64 ; N, 6.27
Found : C, 53.34 ; H, 5.62 ; N, 6.04.
C. L. = 40 %

## Example 13

N-[2-[[2-(3,4-Dimethoxyphenyl) ethyl] methylamino] ethyl]-N-(1-methylethyl)-3,4-dichlorobenzenesulphonamide

The title compound was prepared by reaction of N-β-chloroethyl-N-(1-methylethyl)-3,4-dichlorobenzenesulphonamide with N-methylhomoveratrylamine in xylene containing inorganic carbonate acid scavengers by heating for five days.

Analysis for : $C_{22}H_{30}N_2Cl_2O_4S$
Calculated : C, 53.99 ; H, 6.18 ; N, 5.72.
Found : C, 53.94 ; H, 6.12 ; N, 5.74.
C. L. = 0 %

Example 14

3,4-Dichloro-N-[4-[[2-(3,4-dimethoxyphenyl) ethyl] methylamino] butyl]-N-(1-methylethyl) benzenesulphonamide

3,4-Dichlorobenzene sulphonyl chloride (98.3 g) (0.4 m) in methylene chloride (200 ml) was added dropwise to 4-amino-1-butanol (35.69 g) (0.4 m) in methylene chloride (500 ml). After workup crude 3,4-dichloro-N-(4-hydroxybutyl) benzene sulphonamide is obtained as a gummy white crystalline solid, m. p. 78-80 °C. The compound was identified by combustion and spectral data. 3,4-dichloro-N-(4-hydroxybutyl)benzenesulphonamide (40.10 g) (0.134 m) in dry dimethylformamide (400 ml) was treated with NaH/mineral oil/60 % (5.38 g) (0.134 m) and the reaction mixture was stirred one hour at room temperature. Then 2-bromopropane (16.5 g) (0.134 m) was added and the reaction mixture heated at a temperature of 35 to 50 °C over the weekend. The reaction mixture was worked up and the crude product chromatographed on dry column alumina (1 kg) with 1 to 1 ethyl acetate/hexane to provide 3,4-dichloro-N-(4-hydroxybutyl)-N-(1-methylethyl)benzenesulphonamide (16.09 g) as a light yellow oil. Pyridine-chromic acid complex was prepared by adding chromium trioxide (26.4 g) (94 m. moles) to pyridine (41.76 g) in methylene chloride (660 ml).

The reaction mixture was stirred at room temperature for 3/4 hours. Then, 3,4-dichloro-N-(4-hydroxybutyl)-N-(1-methylethyl) benzenesulphonamide (15.01 g) in methylene chloride (150 ml) was added all at once. After 1/2 hour the liquid was decanted and washed with 5 % aqueous NaOH, 5 % aqueous HCl, 5 % aqueous $NaHCO_3$ and finally brine. After removal of solvent there was obtained 3,4-dichloro-N-(1-methylethyl)-N-(4-oxobutyl)benzenesulphonamide as a dark oil (9.01 g) which started to crystallise nicely but was used below without further purification. 3,4-Dichloro-N-(1-methylethyl)-N-(4-oxobutyl)benzenesulphonamide (8.90 g) ( .027 m) and N-methyl-homoveratrylamine (5.29 g) ( .027 m) in absolute ethanol (100 ml) containing $PtO_2$ (0.6 g) was shaken under hydrogen (7 hours) and the reaction mixture was filtered and stripped to provide the crude product (13.32 g) as a dark greenish gum. This material was chromatographed, after removal of a small amount of insoluble solid, on dry column silica gel (500 gm) with 30 % methanol/70 % ethyl acetate to provide the title compound (7.26 g) as a clear white gum.

Analysis for : $C_{24}H_{34}N_2Cl_2O_4S$
Calculated : C, 55.70 ; H, 6.62 ; N, 5.41.
Found : C, 55.63 ; H, 6.59 ; N, 5.29.
P. R. : = 68 % ;
C. L. : = 0 %.

Example 15

N-[3-[[2-(3,4-dimethoxyphenyl)ethyl]methylamino]propyl]-N-(1-methylethyl)-2,3,4-trimethoxybenzenesulphonamide

2,3,4-trimethoxybenzenesulphonyl chloride (20.4 g) (0.765 m) in methylene chloride (100 ml) was added dropwise to 3-aminopropanol (17.21 g) in methylene chloride (300 ml). After stirring overnight the reaction mixture was washed with dilute aqueous HCl, dried and stripped to provide a solid. Trituration with diethyl ether provided N-(3-hydroxypropyl)-2,3,4-trimethoxybenzenesulphonamide (18.0 g) m. r. 95-97 °C.

Analysis for : $C_{12}H_{19}NO_6S$
Calculated : C, 47.20 ; H, 6.27 ; N, 4.57.
Found : C, 46.89 ; H, 6.16 ; N, 4.57.

N-(3-hydroxypropyl)-2,3,4-trimethoxybenzenesulphonamide (17.79 g) ( 0.58 m) in dry dimethylformamide (100 ml) was treated with 60 % NaH/mineral oil (2.31 g) ( .058 m) and then 2-bromopropane (2.31 g) ( .058 m) and heated with stirring overnight. Chromatography indicated only 50 % reaction. Therefore the crude product was retreated using the same quantities to provide a crude product (23.84 g). Chromatography provided an alcohol, N-(3-hydroxypropyl)-2,3,4-trimethoxy-N-(1-methylethyl)benzenesulphonamide, (10.92 g) containing a trace of ethyl acetate. The alcohol was oxidised to the aldehyde, 2,3,4-trimethoxy-N-(1-methylethyl)-N-(3-oxopropyl)benzenesulphonamide, by use of the $CrO_3$/pyridine reagent, prepared from $CrO_3$ (18.84 g), pyridine (29.79 g) in methylene chloride (500 ml). The aldehyde was isolated as a dark gum (7.70 g). The aldehyde (7.50 g) (.0384 m), N-methyl-homoveratrylamine (6.83 g) (0.350 m) in absolute ethanol (100 ml) containing $PtO_2$ (0.5 g) was shaken under hydrogen all day. The crude product (8.01 g) obtained from this procedure was subjected to chromatography to give the title compound as the pure product, a white gum (5.71 g).

Analysis for : $C_{20}H_{40}N_2O_7S$
Calculated : C, 59.52 ; H, 7.68 ; N, 5.34.
Found : C, 59.29 ; H, 7.59 ; N, 5.20.
P. R. : = 80 % ;
C. L. : = 0 %.

**Claims** (for the Contracting States : BE, CH, DE, FR, GB, IT, LI, LU, NL, SE)

1. A compound having the formula :

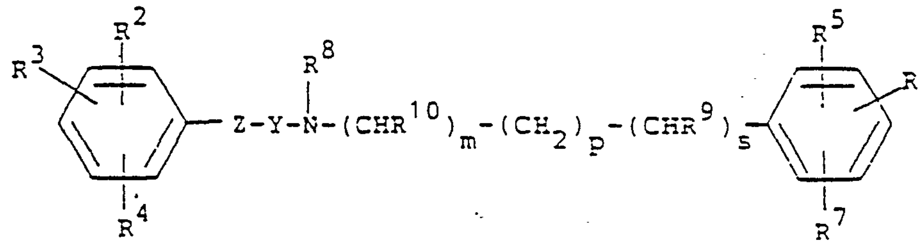

in which

Y is $-(CH_2)_n-$ where n is 1, 2, 3 or 4 or

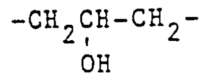

Z is

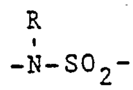

or

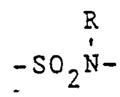

where R is hydrogen, alkyl of 1-6 carbon atoms, polyfluorinated alkyl of 1 to 6 carbon atoms, —CN, cyanoalkyl in which the alkyl moiety contains 1 to 3 carbon atoms, cycloalkyl of 3 to 6 carbon atoms or cycloalkylalkyl of 4 to 8 carbon atoms ;

$R^2$, $R^3$, $R^4$, $R^5$, $R^6$ and $R^7$, independently, are hydrogen, alkoxy of 1 to 3 carbon atoms, trifluoromethyl, —Cl, —Br or —F ;

$R^8$ is hydrogen or alkyl of 1 to 6 carbon atoms ;

$R^9$ is alkyl of 1 to 3 carbon atoms ;

$R^{10}$ is alkyl of 1 to 3 carbon atoms ;

m is 0 or 1 ;

p is 0, 1, 2 or 3 ; and

s is 0 or 1 ;

or a pharmaceutically acceptable salt thereof, with the provisos that the sum of m, p and s must be 1, 2, 3 or 4 ; and the number of linear atoms in the bridge connecting the two aromatic rings does not exceed 9 ; and when n is 1 or 2, two of $R^2$, $R^3$ and $R^4$ are other than hydrogen.

2. A compound having the formula :

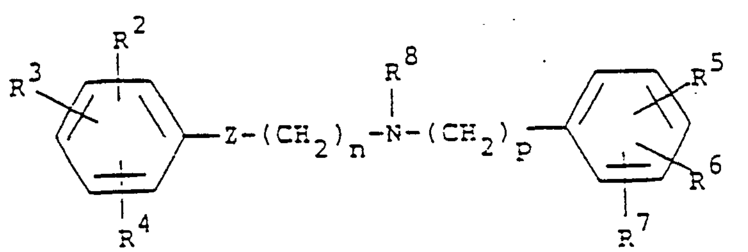

(Ie)

in which

Z represents

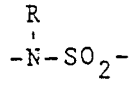

or

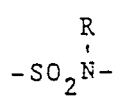

where R is alkyl of 1 to 4 carbon atoms or cycloalkyl of 3 to 6 carbon atoms ;

$R^2$, $R^3$ and $R^4$ are, independently, hydrogen, alkoxy of 1 to 3 carbon atoms, trifluoromethyl, —Cl, —Br or —F ;

$R^5$, $R^6$ and $R^7$ are, independently, hydrogen, alkoxy of 1 to 3 carbon atoms, trifluoromethyl, —Cl, —Br or —F ;

n is 2, 3 or 4, with the proviso that when n is 2, two of $R^2$, $R^3$ and $R^4$ are other than hydrogen ;

p is 1, 2 or 3 ; and

$R^8$ is alkyl of 1 to 3 carbon atoms ;

or a pharmaceutically acceptable salt thereof.

3. A compound having the formula :

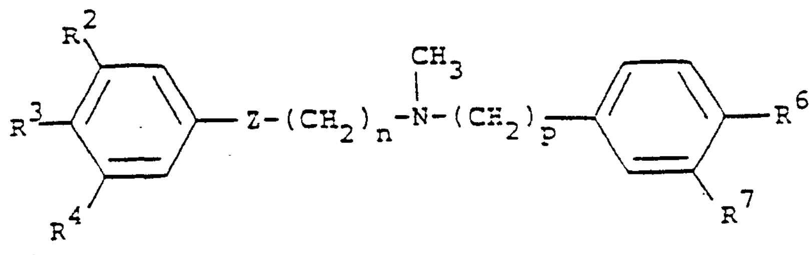

(Ia)

in which

Z represents

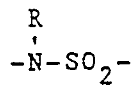

or

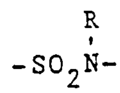

where R is alkyl of 1 to 4 carbon atoms ;

$R^2$ is hydrogen or alkoxy of 1 to 3 carbon atoms ;

$R^3$, $R^4$, $R^6$ and $R^7$ are, independently, alkoxy of 1 to 3 carbon atoms, trifluoromethyl, —Cl, —Br or —F ;

n is 2, 3 or 4 ; and

p is 1 or 2 ;

or a pharmaceutically acceptable salt thereof.

4. A compound having the formula :

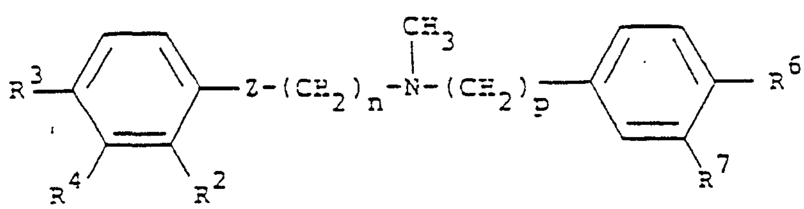

(Ic)

in which

Z represents

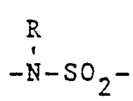

or

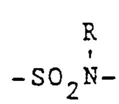

where R is alkyl of 1 to 4 carbon atoms ;

$R^2$ is hydrogen or alkoxy of 1 to 3 carbon atoms ;

$R^3$, $R^4$, $R^6$ and $R^7$ are, independently, alkoxy of 1 to 3 carbon atoms, trifluoromethyl, —Cl, —Br or —F ;

n is 2, 3 or 4 ; and

p is 1 or 2 ;

or a pharmaceutically acceptable salt thereof.

5. A compound having the formula :

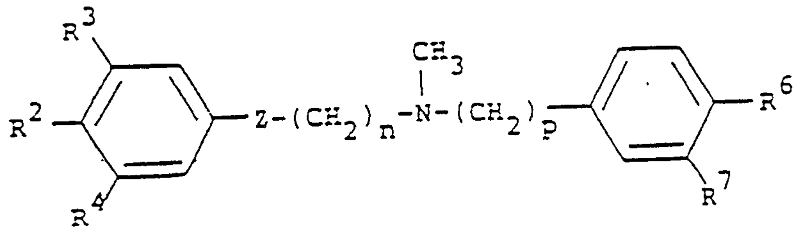

(Ib)

in which

Z represents

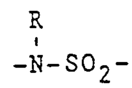

or

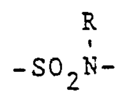

where R is alkyl of 1 to 4 carbon atoms ;

$R^2$ is hydrogen or alkoxy of 1 to 3 carbon atoms ;

$R^3$, $R^4$, $R^6$ and $R^7$ are, independently, alkoxy of 1 to 3 carbon atoms, trifluoromethyl, —Cl, —Br or —F ;

n is 2, 3 or 4 ; and

p is 1 or 2 ;

or a pharmaceutically acceptable salt thereof.

6. A compound having the formula :

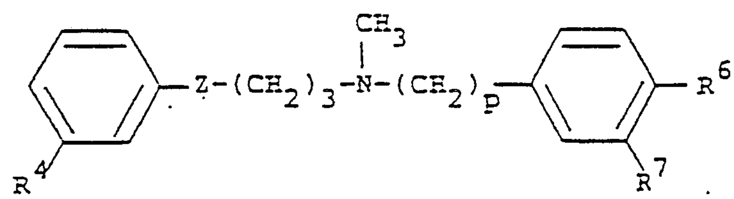

(Id)

in which

Z represents

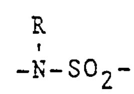

or

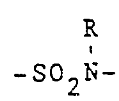

where R is alkyl of 1 to 4 carbon atoms ;

$R^4$, $R^6$ and $R^7$ are, independently, alkoxy of 1 to 3 carbon atoms, trifluoromethyl, —Cl, —Br or —F ; and

p is 1 or 2 ;

or a pharmaceutically acceptable salt thereof.

7. 3,4-Dichloro-N-[4-[[2-(3,4-dimethoxyphenyl)ethyl]methylamino]butyl]-N-(1-methylethyl)benzene sulphonamide or a pharmaceutically acceptable salt thereof.

8. N-[3-[[2-(3,4-dimethoxyphenyl)ethyl]methylamino]propyl]-N-(1-methylethyl)-2,3,4-trimethoxybenzene-sulphonamide or a pharmaceutically acceptable salt thereof.

9. 3-[[2-(3,4-Dimethoxyphenyl)ethyl]methylamino]-N-(1-methylethyl)-N-(3,4,5-trimethoxyphenyl)-1-propanesulphonamide or a pharmaceutically acceptable salt thereof.

10. 3,4-Dichloro-N-[3-[[2-(3,4-dimethoxyphenyl)ethyl]methylamino]propyl]-N-(1-methylethyl)benzenesulphonamide or a pharmaceutically acceptable salt thereof.

11. A compound selected from N-(3,5-dichlorophenyl)-3-[[2-(3,4-dimethoxyphenyl)ethyl]methylamino]-N-(1-methylethyl)-1-propanesulphonamide ; N-(3-trifluoromethylphenyl)-3-[[2-(3,4-dimethoxyphenyl)ethyl]methylamino]-1-propanesulphonamide ; N-(3-trifluoromethylphenyl)-3-[[2-(3,4-dimethoxyphenyl)ethyl]methylamino]-N-(1-methylethyl)-1-propanesulphonamide ; N-[3-[[2-(3,4-dimethoxyphenyl)ethyl]methylamino]propyl]-N-(1-methyethyl)-3-(trifluoromethyl)benzenesulphonamide ; N-(3,4-dichlorophenyl)-3-[[2-(3,4-dimethoxyphenyl)ethyl]methylamino]-N-(1-methylethyl)-1-propanesulphonamide ; N-(3,4,5-trimethoxyphenyl)-3-[[1-(3,4,5-trimethoxyphenyl)ethyl]amino]-1-propanesulphonamide ; N-(3,5-dimethoxyphenyl)-3-[[2-(3,4-dimethoxyphenyl)ethyl]methylamino]-N-(1-methylethyl)-1-propanesulphonamide ; N-(3,4-dimethoxyphenyl)-3-[[2-(3,4-dimethoxyphenyl)ethyl]methylamino]-N-(1-methylethyl)-1-propanesulphonamide ; 3-[[(3,4-Dimethoxyphenyl)methyl]methylamino]-N-[3-(trifluoromethyl)phenyl]-1-propanesulphonamide ; N-[2-[[2-(3,4-dimethoxyphenyl)ethyl]methylamino]ethyl]-N-(1-methylethyl)-3,4-dichlorobenzenesulphonamide ; N-(3,5-dimethoxyphenyl)-3-[[2-(3,4-dimethoxyphenyl)ethyl]methylamino]-1-propanesulphonamide ; N-(3,4-dimethoxyphenyl)-3-[[2-(3,4-dimethoxyphenyl)ethyl]methylamino]-1-propanesulphonamide ; 3-[[2-(3,4-Dimethoxyphenyl)ethyl]methylamino]-N-(3,4,5-trimethoxyphenyl)-1-propanesulphonamide ; N-(3,5-dichlorophenyl)-3-[[2-(3,4-dimethoxyphenyl)ethyl]methylamino]-1-propanesulphonamide ; N-(3,4-dichlorophenyl)-3-[[2-(3,4-dimethoxyphenyl)ethyl]methylamino]-1-propanesulphonamide ; N-[2-[[2-(3,4-dimethoxyphenyl)ethyl]methylamino]ethyl]-3,4-dichlorobenzenesulphonamide and the pharmaceutically acceptable salts thereof.

12. A compound as claimed in any one of Claims 1 to 11, for use as a pharmaceutical.

13. A pharmaceutical composition comprising a compound as claimed in any one of Claims 1 to 11 in association or combination with a pharmaceutically acceptable carrier.

14. Use of a compound as claimed in any one of Claims 1 to 11 for the preparation of a medicament for use as an anti-arrhythmic agent.

15. A process for the preparation of a compound as claimed in Claim 1, which comprises

(1) sulphonylation of an aromatic amine having the formula Ar—NHR (wherein R is as defined in Claim 1 and Ar is a group having the formula XIX

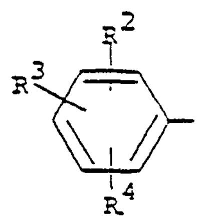

(XIX)

wherein $R^2$, $R^3$ and $R^4$ are as defined in Claim 1 to introduce the sulphonyl group having the formula II

$$—SO_2—Y^1—NR^8—CHA^1A^2$$

(wherein $Y^1$ is the same as Y save that if an hydroxy group is present in Y the hydroxy group may be protected by a removable protecting group ; $R^8$ is as defined in Claim 1, $A^1$ represents hydrogen or alkyl of 1 to 3 carbon atoms and $A^2$ represents such a group that $A^1A^2CH—$ is the same as formula XX

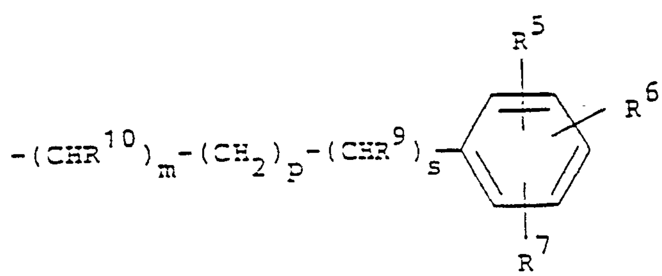

(XX)

[where $R^5$, $R^6$, $R^7$, $R^9$, $R^{10}$, m, p and s are as defined in Claim 1]) ; or

(2) sulphonylation of an amine having the formula III

$$H\overset{*}{N}R—Y^1—NR^8—CHA^1A^2 \qquad (III)$$

(where $A^1$, $A^2$, R, $R^8$ and Y are as defined above) to introduce a sulphonyl group having the formula $Ar—SO_2—$ (where Ar is as defined above) at the nitrogen atom marked with an asterisk ; or

(3) alkylation of a sulphonamide having the formula

$$Ar—SO_2—NHR \quad (IV)$$

(where Ar and R are as defined above) to introduce a substituted alkyl group having the formula V

$$—Y^1—NR^8—CHA^1A^2 \quad (V)$$

(where $A^1$, $A^2$, $R^8$ and $Y^1$ are as defined above) ; or

(4) alkylation of an amine having the formula VI

$$Ar—Z—Y^1—\overset{*}{N}HR^8 \quad (VI)$$

(where Z is as defined in Claim 1 and Ar, $R^8$ and $Y^1$ are as defined above) to introduce a substituted alkyl group having the formula $—CHA^1A^2$ (where $A^1$ and $A^2$ are as defined above) at the nitrogen atom marked with an asterisk ; or

(5) reductive amination of a carbonyl compound having the formula

$$A^1A^2C{=}O \quad (VII)$$

(where $A^1$ and $A^2$ are as defined above) with an amine having the formula VI as illustrated above ; or

(6) reduction of a Schiff's base having the formula VIII or IX

$$Ar—Z—Y^1—N{=}CA^1A^2 \quad (VIII)$$

$$Ar—Z—(CH_2)_q—C{=}N—CHA^1A^2 \quad (IX)$$

(where $A^1$, $A^2$, Ar, $Y^1$ and Z are as defined above and q is 1, 2 or 3) or of an amide or thioamide having formula X or XI

$$Ar—Z—Y^1—NR^8—CX—A^2 \quad (X)$$

$$Ar—Z—(CH_2)_q—CX—NR^8—CHA^1A^2 \quad (XI)$$

(where $A^1$, $A^2$, Ar, $R^8$, $Y^1$, Z and q are as defined above and X is O or S) ; or

(7) alkylation of an amine having the formula XII

$$HNR^8—CHA^1A^2 \quad (XII)$$

(where $A^1$, $A^2$ and $R^8$ are as defined above) to introduce a substituted alkyl group having the formula XIII

$$Ar—Z—Y^1— \quad (XIII)$$

(where Ar, $Y^1$ and Z are as defined above) ; or

(8) reductive amination of an aldehyde having the formula XIV

$$Ar—Z—(CH_2)_q—CHO \quad (XIV)$$

(where Ar, Z and q are as defined above) with an amine having the formula XII as illustrated above ; or

(9) alkylation of a sulphonamide having the formula XV or XVI

$$Ar—SO_2—\overset{*}{N}H—Y^1—NR^8—CHA^1A^2 \quad (XV)$$

$$Ar—\overset{*}{N}H—SO_2—Y^1—NR^8—CHA^1A^2 \quad (XVI)$$

(where $A^1$, $A^2$, Ar, $R^8$ and $Y^1$ are as defined above) to introduce an alkyl or substituted alkyl group $R^{11}$ (where $R^{11}$ is any meaning of R given in Claim 1 except hydrogen) at the nitrogen atom marked with an asterisk ; or

(10) reductive amination of an alkanone containing 1 to 6 carbon atoms with an amine having the formula XVII

$$Ar—Z—Y^1—NH—CHA^1A^2 \quad (XVII)$$

(where $A^1$, $A^2$, Ar, $Y^1$ and Z are as defined above) ; or

(11) reduction of an amide or thioamide having the formula XVIII

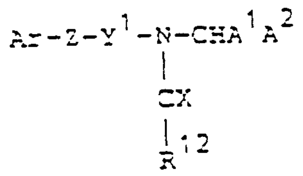

(XVIII) .

(where $A^1$, $A^2$, Ar, X, $Y^1$ and Z are as defined above and $R^{12}$ is hydrogen or alkyl of 1 to 5 carbon atoms) and, where appropriate, removal of a removable protecting group and, if desired, removal of the acid from an acid addition salt to form the free base, metathetical displacement to convert one salt into another or neutralisation of the free base to form a salt.

**Claims** (for the Contracting State AT)

1. A process for the preparation of a compound having the formula :

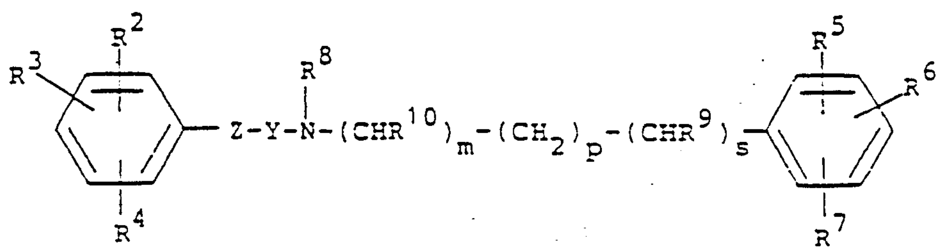

(I)

in which

Y is $—(CH_2)_n—$ where n is 1, 2, 3 or 4 or

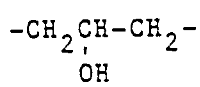

Z is

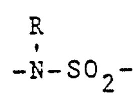

or

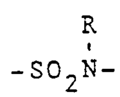

where R is hydrogen, alkyl of 1 to 6 carbon atoms, polyfluorinated alkyl of 1 to 6 carbon atoms, —CN, cyanoalkyl in which the alkyl moiety contains 1 to 3 carbon atoms, cycloalkyl of 3 to 6 carbon atoms or cycloalkylalkyl of 4 to 8 carbon atoms ;

$R^2$, $R^3$, $R^4$, $R^5$, $R^6$ and $R^7$, independently, are hydrogen, alkoxy of 1 to 3 carbon atoms, trifluoromethyl, —Cl, —Br or —F ;

$R^8$ is hydrogen or alkyl of 1 to 6 carbon atoms ;

$R^9$ is alkyl of 1 to 3 carbon atoms ;

$R^{10}$ is alkyl of 1 to 3 carbon atoms ;

m is 0 or 1 ;

p is 0, 1, 2 or 3 ; and

s is 0 or 1 ;

or a pharmaceutically acceptable salt thereof, with the provisos that the sum of m, p and s must be 1, 2, 3 or 4 ; and the number of linear atoms in the bridge connecting the two aromatic rings does not exceed 9 ; and when n is 1 or 2, two of $R^2$, $R^3$ and $R^4$ are other than hydrogen, which comprises :

(1) sulphonylation of an aromatic amine having the formula Ar—NHR (wherein R is as defined above and Ar is a group having the formula XIX

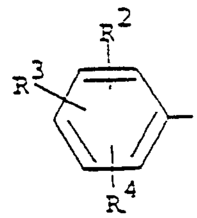

(XIX)

wherein $R^2$, $R^3$ and $R^4$ are as defined above) to introduce the sulphonyl group having the formula II

$$—SO_2—Y^1—NR^8—CHA^1A^2$$

(wherein $Y^1$ is the same as Y as defined above save that if an hydroxy group is present in Y the hydroxy group may be protected by a removable protecting group ; $R^8$ is as defined above, $A^1$ represents hydrogen or alkyl of 1 to 3 carbon atoms and $A^2$ represents such a group that $A^1A^2CH—$ is the same as formula XX

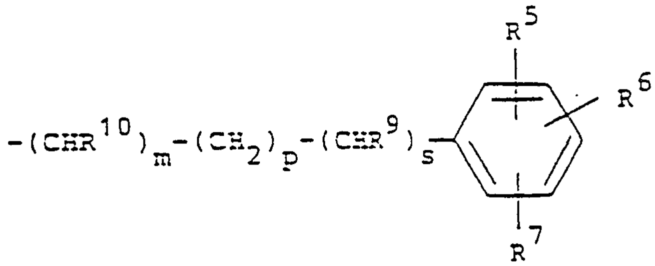

(XX)

[where $R^5$, $R^6$, $R^7$, $R^9$, $R^{10}$, m, p and s are as defined above]) ; or

(2) sulphonylation of an amine having the formula III

$$H\overset{*}{N}R—Y^1—NR^8—CHA^1A^2 \quad (III)$$

(where $A^1$, $A^2$, R, $R^8$ and Y are as defined above) to introduce a sulphonyl group having the formula $Ar—SO_2—$ (where Ar is as defined above) at the nitrogen atom marked with an asterisk ; or

(3) alkylation of a sulphonamide having the formula

$$Ar—SO_2—NHR \quad (IV)$$

(where Ar and R are as defined above) to introduce a substituted alkyl group having the formula V

$$—Y^1—NR^8—CHA^1A^2 \quad (V)$$

(where $A^1$, $A^2$, $R^8$ and $Y^1$ are as defined above) ; or

(4) alkylation of an amine having the formula VI

$$Ar—Z—Y^1—\overset{*}{N}HR^8 \quad (VI)$$

(where Ar, $R^8$, $Y^1$ and Z are as defined above) to introduce a substituted alkyl group having the formula $—CHA^1A^2$ (where $A^1$ and $A^2$ are as defined above) at the nitrogen atom marked with an asterisk ; or

(5) reductive amination of a carbonyl compound having the formula

$$A^1A^2C{=}O \quad (VII)$$

(where $A^1$ and $A^2$ are as defined above) with an amine having the formula VI as illustrated above ; or

(6) reduction of a Schiff's base having the formula VIII or IX

$$Ar—Z—Y^1—N{=}CA^1A^2 \quad (VIII)$$

$$Ar—Z—(CH_2)_q—C{=}N—CHA^1A^2 \quad (IX)$$

(where $A^1$, $A^2$, Ar, $Y^1$ and Z are as defined above and q is 1, 2 or 3) or of an amide or thioamide having formula X or XI

$$Ar—Z—Y^1—NR^8—CX—A^2 \quad (X)$$

$$Ar—Z—(CH_2)_q—CX—NR^8—CHA^1A^2 \qquad (XI)$$

(where $A^1$, $A^2$, Ar, $R^8$, $Y^1$, Z and q are as defined above and X is O or S) ; or

(7) alkylation of an amine having the formula XII

$$HNR^8—CHA^1A^2 \qquad (XII)$$

(where $A^1$, $A^2$ and $R^8$ are as defined above) to introduce a substituted alkyl group having the formula XIII

$$Ar—Z—Y^1— \qquad (XIII)$$

(where Ar, $Y^1$ and Z are as defined above) ; or

(8) reductive amination of an aldehyde having the formula XIV

$$Ar—Z—(CH_2)_q—CHO \qquad (XIV)$$

(where Ar, Z and q are as defined above) with an amine having the formula XII as illustrated above ; or

(9) alkylation of a sulphonamide having the formula XV or XVI

$$Ar—SO_2—\overset{*}{N}H—Y^1—NR^8—CHA^1A^2 \qquad (XV)$$

$$Ar—\overset{*}{N}H—SO_2—Y^1—NR^8—CHA^1A^2 \qquad (XVI)$$

(where $A^1$, $A^2$, Ar, $R^8$ and $Y^1$ are as defined above) to introduce an alkyl or substituted alkyl group $R^{11}$ (where $R^{11}$ is any meaning of R given above except hydrogen) at the nitrogen atom marked with an asterisk ; or

(10) reductive amination of an alkanone containing 1 to 6 carbon atoms with an amine having the formula XVII

$$Ar—Z—Y^1—NH—CHA^1A^2 \qquad (XVII)$$

(where $A^1$, $A^2$, Ar, $Y^1$ and Z are as defined above) ; or

(11) reduction of an amide or thioamide having the formula XVIII

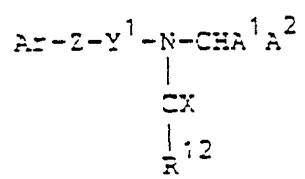

(XVIII)

(where $A^1$, $A^2$, Ar, X, $Y^1$ and Z are as defined above and $R^{12}$ is hydrogen or alkyl or 1 to 5 carbon atoms) and, where appropriate, removal of a removable protecting group and, if desired, removal of the acid from an acid addition salt to form the free base, metathetical displacement to convert one salt into another or neutralisation of the free base to form a salt.

2. A process for the preparation of a compound having the formula I

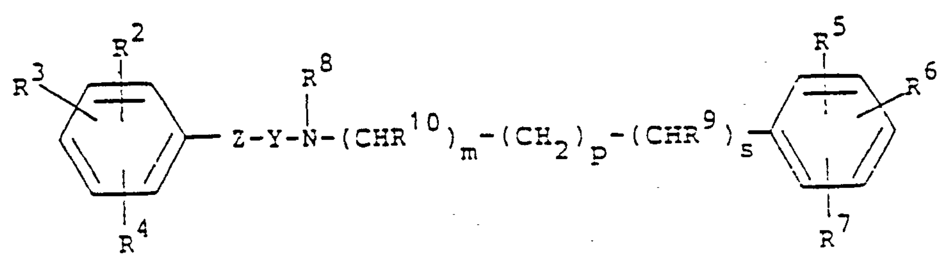

(I)

(where Y, n, Z, R, $R^2$, $R^3$, $R^4$, $R^5$, $R^6$, $R^7$, $R^8$, $R^9$, $R^{10}$, m, p and s are as defined in Claim 1) which comprises removal of acid from an acid addition salt of a compound having the said formula I.

3. A process for the preparation of a pharmaceutically acceptable salt of a compound having the formula I

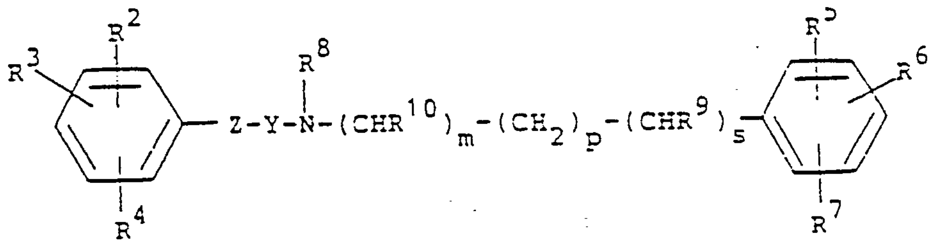

(I)

(where Y, n, Z, R, $R^2$, $R^3$, $R^4$, $R^5$, $R^6$, $R^7$, $R^8$, $R^9$, $R^{10}$, m, p and s are as defined in Claim 1), which comprises metathetical displacement to convert another salt of the compound having the formula I into the aforesaid pharmaceutically acceptable salt or neutralisation of the free base form of the compound having the formula I.

4. A process as claimed in any one of Claims 1 to 3, wherein the compound having the formula I or a pharmaceutically acceptable salt thereof is prepared for use as a pharmaceutical.

5. A process as claimed in any one of Claims 1 to 4, wherein Y is $-(CH_2)_n-$ where n is 2, 3 or 4 ; Z represents

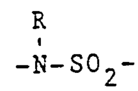

or

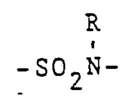

where R is alkyl of 1 to 4 carbon atoms or cycloalkyl of 3 to 6 carbon atoms ; $R^8$ is alkyl of 1 to 3 carbon atoms ; m and s are both 0 and p is 1, 2, or 3.

6. A process as claimed in any one of Claims 1 to 3, wherein a compound having one of the formula Ia, Ib, Ic and Id

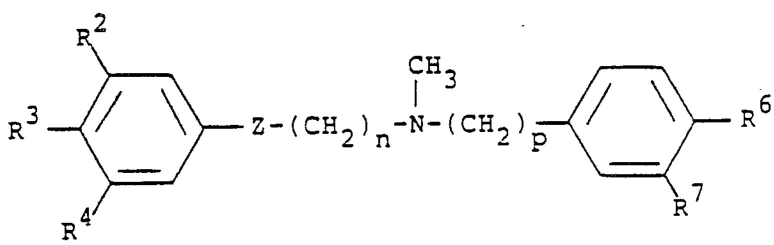

(Ia)

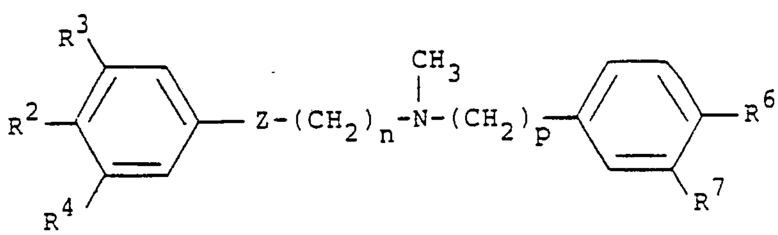

(Ib)

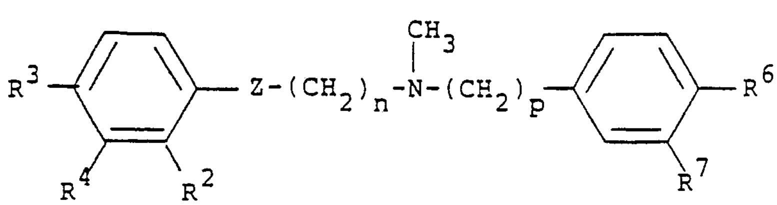

(Ic)

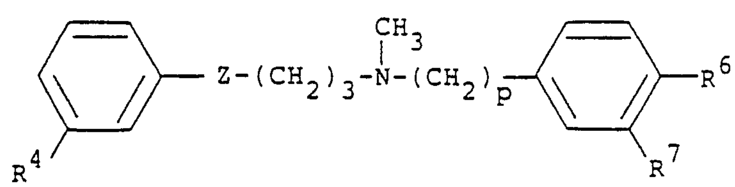

(Id)

in which

Z represents

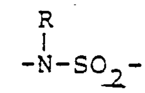

or

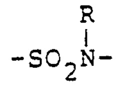

where R is alkyl of 1 to 4 carbon atoms ;

$R^2$ is hydrogen or alkoxy of 1 to 3 carbon atoms ;

$R^3$, $R^4$, $R^6$ and $R^7$ are, independently, alkoxy of 1 to 3 carbon atoms, trifluoromethyl, —Cl, —Br or —F ;

n is 2, 3 or 4 and p is 1 or 2 ;

or a pharmaceutically acceptable salt thereof is prepared.

7. A process as claimed in any one of Claims 1 to 3, wherein a compound selected from 3,4-dichloro-N-[4-[[2-(3,4-dimethoxyphenyl)ethyl]methylamino]butyl]-N-(1-methylethyl)benzenesulphonamide ; N-[3-[[2-(3,4-dimethoxyphenyl)ethyl]methylamino]propyl]-N-(1-methylethyl)-2,3,4-trimethoxybensenesulphonamide ; 3-[[2-(3,4-dimethoxyphenyl)ethyl]methylamino]-N-(1-methylethyl)-N-(3,4,5-trimethoxyphenyl)-1-propanesulphonamide ; 3,4-dichloro-N-[3-[[2-(3,4-dimehoxyphenyl)ethyl]methylamino]propyl]-N-(1-methylethyl)benzenesulphonamide and their pharmaceutically acceptable salts is prepared.

8. A process as claimed in any one of Claims 1 to 3, wherein a compound selected from N-(3,5-dichlorophenyl)-3-[[2-(3,4-dimethoxyphenyl)ethyl]methylamino]-N-(1-methylethyl)-1-propanesulphonamide ; N-(3-trifluoromethylphenyl)-3-[[2-(3,4-dimethoxyphenyl)ethyl]methylamino]-1-propanesulphonamide ; N-(3-trifluoromethylphenyl)-3-[[2-(3,4-dimethoxyphenyl)ethyl]methylamino]-N-(1-methylethyl)-1-propanesulphonamide ; N-[3-[[2-(3,4-dimethoxyphenyl)ethyl]methylamino]propyl]-N-(1-methylethyl)-3-(trifluoromethyl)benzenesulphonamide ; N-(3,4-dichlorophenyl)-3-[[2-(3,4-dimethoxyphenyl)ethyl]methylamino]-N-(1-methylethyl)-1-propanesulphonamide ; N-(3,4,5-trimethoxyphenyl)-3-[[1-(3,4,5-trimethoxyphenyl)ethyl]amino]-1-propanesulphonamide ; N-(3,5-dimethoxyphenyl)-3-[[2-(3,4-dimethoxyphenyl)ethyl]methylamino]-N-(1-methylethyl)-1-propanesulphonamide ; N-(3,4-dimethoxyphenyl)-3-[[2-(3,4-dimethoxyphenyl)ethyl]methylamino]-N-(1-methylethyl)-1-propanesulphonamide ; 3-[[(3,4-dimethoxyphenyl)methyl]methylamino]-N-[3-trifluoromethyl)phenyl]-1-propanesulphonamide ; N-[2-[[2-(3,4-dimethoxyphenyl)ethyl]methylamino]ethyl]-N-(1-methylethyl)-3,4-dichlorobenzenesulphonamide ; N-(3,5-dimethoxyphenyl)-3-[[2-(3,4-dimethoxyphenyl)ethyl]methylamino]-1-propanesulphonamide ; N-(3,4-dimethoxyphenyl)-3-[[2-(3,4-dimethoxyphenyl)ethyl]methylamino]-1-propanesulphonamide ; 3-[[2-(3,4-dimethoxyphenyl)ethyl]methylamino]-N-(3,4,5-trimethoxyphenyl)-1-propanesulphonamide ; N-(3,5-dichlorophenyl)-3-[[2-(3,4-dimethoxyphenyl)ethyl]methylamino]-1-propanesulphonamide ; N-(3,4-dichlorophenyl)-3-[[2(3,4-dimethoxyphenyl)ethyl]methylamino]-1-propanesulphonamide ; N-[2-[[2-(3,4-dimethoxyphenyl)ethyl]methylamino]ethyl]-3,4-dichlorobenzenesulphonamide and the pharmaceutically acceptable salts thereof is prepared.

9. A process for the preparation of a pharmaceutical composition in which a compound having formula I as illustrated and defined in Claim 1 or a pharmaceutically acceptable salt thereof is brought into association or combination with a pharmaceutically acceptable carrier.

10. Use of a compound having formula I as illustrated and defined in Claim 1 or a pharmaceutically acceptable salt thereof for the preparation of a medicament for use as an anti-arrhythmic agent.

**Patentansprüche** (für die Vertragsstaaten : BE, CH, DE, FR, GB, IT, LI, LU, NL, SE)

1. Eine Verbindung der Formel

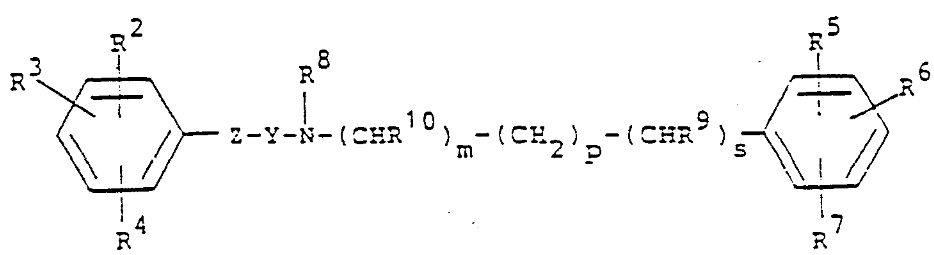

worin

Y —($CH_2$)—, wobei n 1, 2, 3 oder 4 ist, oder

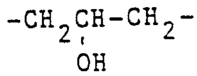

ist ;

Z die Bedeutung

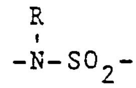

oder

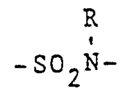

hat, wobei R Wasserstoff, Alkyl mit 1 bis 6 C-Atomen, polyfluoriertes Alkyl mit 1 bis 6 C-Atomen, —CN, Cyanoalkyl, worin der Alkylteil 1 bis 3 C-Atome enthält, Cycloalkyl mit 3 bis 6 C-Atomen oder Cycloalkylalkyl mit 4 bis 8 C-Atomen darstellt ;

$R^2$, $R^3$, $R^4$, $R^5$, $R^6$ und $R^7$ unabhängig voneinander Wasserstoff, Alkoxy mit 1 bis 3 C-Atomen, Trifluormethyl, —Cl, —Br oder —F bedeuten ;

$R^8$ Wasserstoff oder Alkyl mit 1 bis 6 C-Atomen ist ;

$R^9$ Alkyl mit 1 bis 3 C-Atomen darstellt ;

$R^{10}$ die Bedeutung Alkyl mit 1 bis 3 C-Atomen hat ;

m Null oder 1 ist ;

p Null, 1, 2 oder 3 ist ; und

s Null oder 1 ist,

oder ein pharmazeutisch annehmbares Salz hievon, mit den Maßgaben, daß die Summe von m, p und s 1, 2, 3 oder 4 sein muß, und die Zahl der linearen Atome in der die beiden aromatischen Ringe verbindenden Brücke 9 nicht übersteigt, und, wenn n 1 oder 2 ist, zwei von $R^2$, $R^3$ und $R^4$ eine andere Bedeutung als Wasserstoff haben.

2. Eine Verbindung der Formel

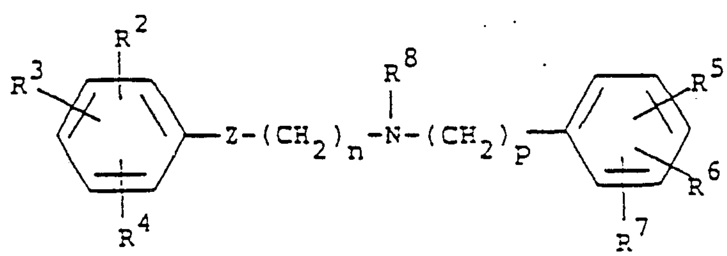

(Ie)

worin

Z

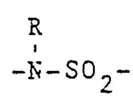

oder

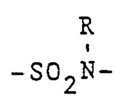

bedeutet, wobei R Alkyl mit 1 bis 4 C-Atomen oder Cycloalkyl mit 3 bis 6 C-Atomen ist ;

$R^2$, $R^3$ und $R^4$ unabhängig voneinander Wasserstoff, Alkoxy mit 1 bis 3 C-Atomen, Trifluormethyl, —Cl, —Br oder —F bedeuten ;

$R^5$, $R^6$ und $R^7$ unabhängig voneinander Wasserstoff, Alkoxy mit 1 bis 3 C-Atomen, Trifluormethyl, —Cl, —Br oder —F sind ;

n 2, 3 oder 4 ist, mit der Maßgabe, daß, wenn n 2 ist, zwei von $R^2$, $R^3$ und $R^4$ eine andere Bedeutung als Wasserstoff haben ;

p 1, 2 oder 3 ist ; und

$R^8$ Alkyl mit 1 bis 3 C-Atomen darstellt,

oder ein pharmazeutisch annehmbares Salz hievon.

3. Eine Verbindung der Formel

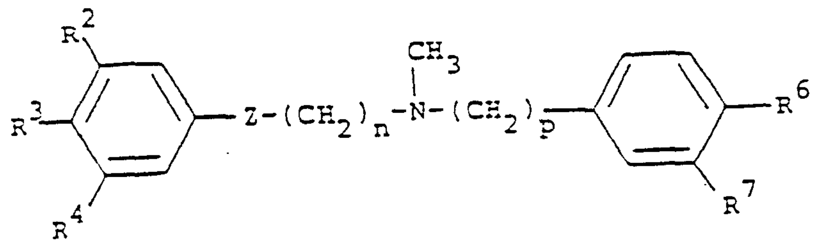

(Ia)

worin

Z

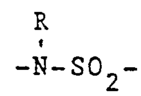

oder

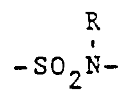

bedeutet, wobei R Alkyl mit 1 bis 4 C-Atomen ist ;

$R^2$ Wasserstoff oder Alkoxy mit 1 bis 3 C-Atomen darstellt ;

$R^3$, $R^4$, $R^6$ und $R^7$ unabhängig voneinander Alkoxy mit 1 bis 3 C-Atomen, Trifluormethyl, —Cl, —Br oder —F bedeuten ;

n 2, 3 oder 4 ist und

p 1 oder 2 ist,

oder ein pharmazeutisch annehmbares Salz hievon.

4. Eine Verbindung der Formel

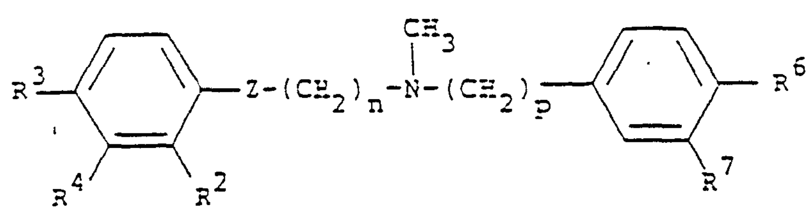

(Ic)

worin

Z

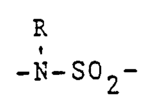

oder

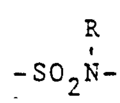

bedeutet, wobei R Alkyl mit 1 bis 4 C-Atomen ist ;

$R^2$ Wasserstoff oder Alkoxy mit 1 bis 3 C-Atomen darstellt ;

$R^3$, $R^4$, $R^6$ und $R^7$ unabhängig voneinander Alkoxy mit 1 bis 3 C-Atomen, Trifluormethyl, —Cl, —Br oder —F bedeuten ;

n 2, 3 oder 4 ist und

p 1 oder 2 ist,

oder ein pharmazeutisch annehmbares Salz hievon.

5. Eine Verbindung der Formel

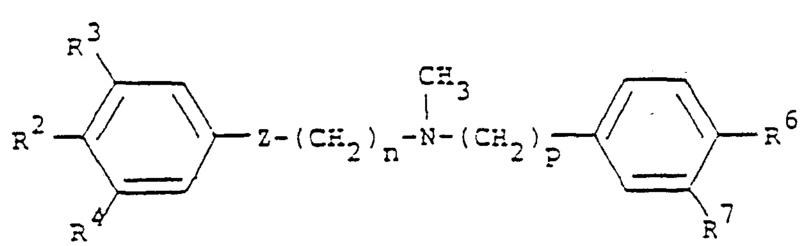

(Ib)

worin
Z

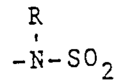

oder

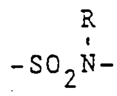

bedeutet, wobei R Alkyl mit 1 bis 4 C-Atomen ist ;
$R^2$ Wasserstoff oder Alkoxy mit 1 bis 3 C-Atomen darstellt ;
$R^3$, $R^4$, $R^6$ und $R^7$ unabhängig voneinander Alkoxy mit 1 bis 3 C-Atomen, Trifluormethyl, —Cl, —Br oder —F sind ;
n 2, 3 oder 4 ist und
p 1 oder 2 ist,
oder ein pharmazeutisch annehmbares Salz hievon.

6. Eine Verbindung der Formel

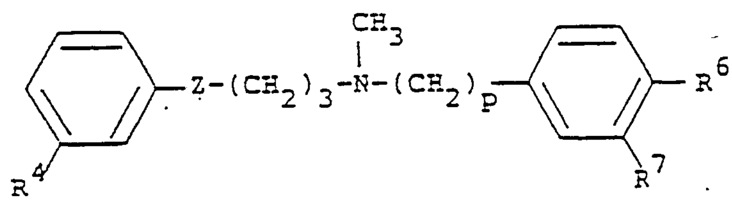

(Id)

worin
Z

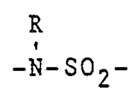

oder

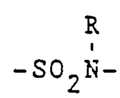

bedeutet, wobei R Alkyl mit 1 bis 4 C-Atomen ist ;
$R^4$, $R^6$ und $R^7$ unabhängig voneinander Alkoxy mit 1 bis 3 C-Atomen, Trifluormethyl, —Cl, —Br oder —F bedeuten ; und
p 1 oder 2 ist,
oder ein pharmazeutisch annehmbares Salz hievon.

7. 3,4-Dichlor-N-[4-[2-(3,4-dimethoxyphenyl)-äthyl]-methylamino]-butyl]-N-(1-methyläthyl)-benzosulfonamid oder ein pharmazeutisch annehmbares Salz hievon.

8. N-[3-[[2-(3,4-Dimethoxyphenyl)-äthyl]-methylamino]-propyl]-N-(1-methyläthyl)-2,3,4-trimethoxybenzolsulfonamid oder ein pharmazeutisch annehmbares Salz hievon.

9. 3-[[2-(3,4-Dimethoxyphenyl)-äthyl]-methylamino]-N-(1-methyläthyl)-N-(3,4,5-trimethoxyphenyl)-1-propansulfonamid oder ein pharmazeutisch annehmbares Salz hievon.

10. 3,4-Dichlor-N-[3-[[2-(3,4-dimethoxyphenyl)-äthyl]-methylamino]-propyl]-N-(1-methyläthyl)-benzosulfonamid oder ein pharmazeutisch annehmbares Salz hievon.

11. Eine Verbindung ausgewählt aus N-(3,5-Dichlorphenyl)-3-[[2-(3,4-dimethoxyphenyl)-äthyl]-methylamino]-N-(1-methyläthyl)-1-propansulfonamid ; N-(3-Trifluormethylphenyl)-3-[[2-(3,4-dimethoxyphenyl)-äthyl]-methylamino]-1-propansulfonamid ; N-(3-Trifluormethylphenyl)-3-[[2-(3,4-dimethoxyphenyl)-äthyl]-methylamino]-N-(1-methyläthyl)-1-propansulfonamid ; N-[3-[[2-(3,4-Dimethoxyphenyl)-äthyl]-methylamino]-propyl]-N-(1-methyläthyl)-3-trifluormethyl)-benzolsulfonamid ; N-(3,4-Dichlorphenyl)-3-[[2-(3,4-dimethoxyphenyl)-äthyl]-methylamino]-N-(1-methyläthyl)-1-propansulfonamid ; N-(3,4,5-Trimethoxyphenyl)-3-[[1-(3,4,5-trimethoxyphenyl)-äthyl]-amino]-1-propansulfonamid ; N-(3,5-Dimethoxyphenyl)-3-[[2-(3,4-dimethoxyphenyl)-äthyl]-methylamino]-N-(1-methyläthyl)-1-propansulfonamid ; N-(3,4-Dimethoxyphenyl)-3-[[2-(3,4-dimethoxyphenyl)-äthyl]-methylamino]-N-(1-methyläthyl)-1-propansulfonamid ; 3-[[3,4-Dimethoxyphenyl)-methyl]-methylamino]-N-[3-(trifluormethyl)-phenyl]-1-propansulfonamid ; N-[2-[[2-(3,4-Dimethoxyphenyl)-äthyl]-methylamino]-äthyl]-N-(1-methyläthyl)-3,4-dich-

lorbenzolsulfonamid ; N-(3,5-Dimethoxyphenyl)-3-[[2-(3,4-dimethoxyphenyl)-äthyl]-methylamino]-1-propansulfonamid ; N-(3,4-Dimethoxyphenyl)-3-[[2-(3,4-dimethoxyphenyl)-äthyl]-methylamino]-1-propansulfonamid ; 3-[[2-(3,4-Dimethoxyphenyl)-äthyl]-methylamino]-N-(3,4,5-trimethoxyphenyl)-1-propansulfonamid ; N-(3,5-Dichlorphenyl)-3-[[2-(3,4-dimethoxyphenyl)-äthyl]-methylamino]-1-propansulfonamid ; N-(3,4-Dichlorphenyl)-3-[[2-(3,4-dimethoxyphenyl)-äthyl]-methylamino]-1-propansulfonamid ; N-[2-[[2-(3,4-Dimethoxyphenyl)-äthyl]-methylamino]-äthyl]-3,4-dichlorbenzolsulfonamid und den pharmazeutisch annehmbaren Salzen hievon.

12. Eine Verbindung, wie in einem der Ansprüche 1 bis 11 beansprucht, zur Verwendung als Pharmazeutikum.

13. Pharmazeutische Zusammensetzung umfassend eine Verbindung, wie in einem der Ansprüche 1 bis 11 beansprucht, in Vereinigung oder Kombination mit einem pharmazeutisch annehmbaren Träger.

14. Verwendung einer Verbindung, wie in einem der Ansprüche 1 bis 11 beansprucht, für die Herstellung eines Medikaments zur Verwendung als antiarrhythmisches Mittel.

15. Verfahren zur Herstellung einer Verbindung, wie in Anspruch 1 beansprucht, welches umfaßt :

(1) Sulfonylierung eines aromatischen Amins der Formel Ar—NHR (worin R wie in Anspruch 1 definiert ist und Ar eine Gruppe der Formel (XIX)

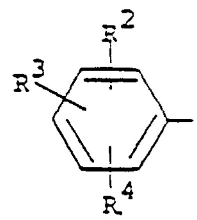

(XIX)

darstellt, worin $R^2$, $R^3$ und $R^4$ wie in Anspruch 1 definiert sind, zum Einführen der Sulfonylgruppe der Formel (II)

$$—SO_2—Y^1—NR^8—CHA^1A^2$$

[worin $Y^1$ die gleiche Bedeutung wie Y hat, vorausgesetzt daß, wenn eine Hydroxygruppe in Y vorhanden ist, die Hydroxygruppe durch eine entfernbare Schutzgruppe geschützt sein kann ; $R^8$ wie in Anspruch 1 definiert ist, $A^1$ Wasserstoff oder Alkyl mit 1 bis 3 C-Atomen darstellt und $A^2$ eine derartige Gruppe ist, daß $A^1A^2CH$— gleich der Formel (XX)

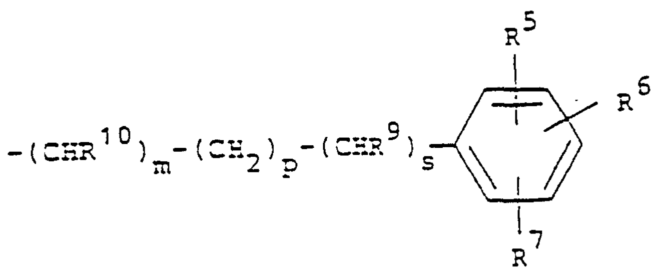

(XX)

(worin $R^5$, $R^6$, $R^7$, $R^9$, $R^{10}$, m, p und s wie in Anspruch 1 definiert sind) ist] oder

(2) Sylfonylierung eines Amins der Formel (III)

$$H\overset{*}{N}R—Y^1—NR^8—CHA^1A^2 \quad (III)$$

(worin $A^1$, $A^2$, R, $R^8$ und Y wie oben definiert sind) zum Einführen einer Sulfonylgruppe der Formel $Ar—SO_2—$ (worin Ar wie oben definiert ist) an dem mit einem Sternchen bezeichneten Stickstoffatom oder

(3) Alkylierung eines Sulfonamids der Formel

$$Ar—SO_2—NHR \quad (IV)$$

(worin Ar und R wie oben definiert sind) zum Einführen einer substituierten Alkylgruppe der Formel (V)

$$—Y^1—NR^8—CHA^1A^2 \quad (V)$$

(worin $A^1$, $A^2$, $R^8$ und $Y^1$ wie oben definiert sind) oder

(4) Alkylierung eines Amins der Formel (VI)

$$Ar{-}Z{-}Y^1{-}\overset{*}{N}HR^8 \qquad (VI)$$

(worin Z wie in Anspruch 1 definiert ist und Ar, $R^8$ und $Y^1$ wie oben definiert sind) zum Einführen einer substituierten Alkylgruppe der Formel $—CHA^1A^2$ (worin $A^1$ und $A^2$ wie oben definiert sind) an dem mit einem Sternchen bezeichneten Stickstoffatom oder

(5) reduktive Aminierung einer Carbonylverbindung der Formel

$$A^1A^2C{=}O \qquad (VII)$$

(worin $A^1$ und $A^2$ wie oben definiert sind) mit einem Amin der Formel (VI), wie oben erläutert, oder

(6) Reduktion einer Schiff'schen Base der Formel (VIII) oder (IX)

$$Ar{-}Z{-}Y^1{-}N{=}CA^1A^2 \qquad (VIII)$$

$$Ar{-}Z{-}(CH_2)_q{-}C{=}N{-}CHA^1A^2 \qquad (IX)$$

(worin $A^1$, $A^2$, Ar, $Y^1$ und Z wie oben definiert sind und q 1, 2 oder 3 ist) oder eines Amids oder Thioamids der Formel (X) oder (XI)

$$Ar{-}Z{-}Y^1{-}NR^8{-}CX{-}A^2 \qquad (X)$$

$$Ar{-}Z{-}(CH_2)_q{-}CX{-}NR^8{-}CHA^1A^2 \qquad (XI)$$

(worin $A^1$, $A^2$, Ar, $R^8$, $Y^1$, Z und q wie oben definiert sind und X die Bedeutung O oder S hat) oder

(7) Alkylierung eines Amins der Formel (XII)

$$HNR^8{-}CHA^1A^2 \qquad (XII)$$

(worin $A^1$, $A^2$ und $R^8$ wie oben definiert sind) zum Einführen einer substituierten Alkylgruppe der Formel (XIII)

$$Ar{-}Z{-}Y^1{-} \qquad (XIII)$$

(worin Ar, $Y^1$ und Z wie oben definiert sind) oder

(8) reduktive Aminierung eines Aldehyds der Formel (XIV)

$$Ar{-}Z{-}(CH_2)_q{-}CHO \qquad (XIV)$$

(worin Ar, Z und q wie oben definiert sind) mit einem Amin der Formel (XII), wie oben erläutert, oder

(9) Alkylierung eines Sulfonamids der Formel (XV) oder (XVI)

$$Ar{-}SO_2{-}\overset{*}{N}H{-}Y^1{-}NR^8{-}CHA^1A^2 \qquad (XV)$$

$$Ar{-}\overset{*}{N}H{-}SO_2{-}Y^1{-}NR^8{-}CHA^1A^2 \qquad (XVI)$$

(worin $A^1$, $A^2$, Ar, $R^8$ und $Y^1$ wie oben definiert sind) zum Einführen einer Alkyl- oder substituierten Alkylgruppe $R^{11}$ (worin $R^{11}$ jede für R in Anspruch 1 angegebene Bedeutung, ausgenommen Wasserstoff, hat) an dem mit einem Sternchen bezeichneten Stickstoffatom oder

(10) reduktive Aminierung eines Alkanons mit 1 bis 6 C-Atomen mit einem Amin der Formel (XVII)

$$Ar{-}Z{-}Y^1{-}NH{-}CHA^1A^2 \qquad (XVII)$$

(worin $A^1$, $A^2$, Ar, $Y^1$ und Z wie oben definiert sind) oder

(11) Reduktion eines Amids oder Thioamids der Formel (XVIII)

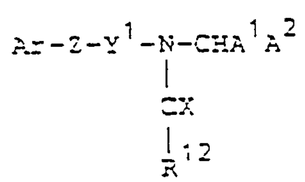

(XVIII)

(worin $A^1$, $A^2$, Ar, X, $Y^1$ und Z wie oben definiert sind und $R^{12}$ Wasserstoff oder Alkyl mit 1 bis 5 C-Atomen ist) und, wenn geeignet, Entfernung einer entfernbaren Schutzgruppe und, wenn gewünscht, Entfernung der Säure von einem Säureadditionssalz zur Bildung der freien Base, metathetisches Verdrängen zum Überführen eines Salzes in ein anderes und Neutralisation der freien Base zur Bildung eines Salzes.

**Patentansprüche** (für den Vertragsstaat AT)

1. Verfahren zur Herstellung einer Verbindung der Formel

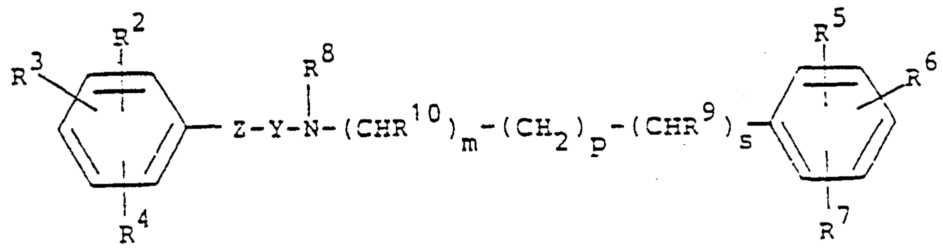

(I)

worin
Y—$(CH_2)$—, wobei n 1, 2, 3 oder 4 ist, oder

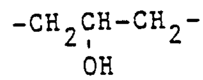

ist ;
Z die Bedeutung

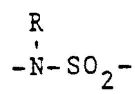

oder

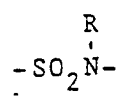

hat, wobei R Wasserstoff, Alkyl mit 1 bis 6 C-Atomen, polyfluoriertes Alkyl mit 1 bis 6 C-Atomen, —CN, Cyanoalkyl, worin der Alkylteil 1 bis 3 C-Atome enthält, Cycloalkyl mit 3 bis 6 C-Atomen oder Cycloalkylalkyl mit 4 bis 8 C-Atomen darstellt ;

$R^2$, $R^3$, $R^4$, $R^5$, $R^6$ und $R^7$ unabhängig voneinander Wasserstoff, Alkoxy mit 1 bis 3 C-Atomen, Trifluormethyl, —Cl, —Br oder —F bedeuten ;

$R^8$ Wasserstoff oder Alkyl mit 1 bis 6 C-Atomen ist ;

$R^9$ Alkyl mit 1 bis 3 C-Atomen darstellt ;

$R^{10}$ die Bedeutung Alkyl mit 1 bis 3 C-Atomen hat ;

m Null oder 1 ist ;

p Null, 1, 2 oder 3 ist ; und

s Null oder 1 ist,

oder eines pharmazeutisch annehmbaren Salzes hievon, mit den Maßgaben, daß die Summe von m, p, und s 1, 2, 3 oder 4 sein muß, und die Zahl von linearen Atomen in der die beiden aromatischen Ringe verbindenden Brücke 9 nicht übersteigt ; und, wenn n 1 oder 2 ist, zwei von $R^2$, $R^3$ und $R^4$ eine andere Bedeutung als Wasserstoff haben, welches umfaßt :

(1) Sulfonylierung eines aromatischen Amins der Formel Ar—NHR (worin R wie oben definiert ist und Ar eine Gruppe der Formel (XIX)

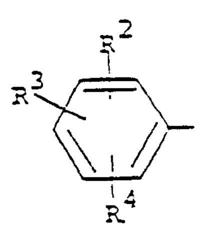

(XIX)

darstellt, worin $R^2$, $R^3$ und $R^4$ wie oben definiert sind) zum Einführen der Sulfonylgruppe der Formel (II)

$$—SO_2—Y^1—NR^8—CHA^1A^2$$

[worin $Y^1$ die gleiche Bedeutung wie Y hat, vorausgesetzt daß, wenn eine Hydroxygruppe in Y vorhanden ist, die Hydroxygruppe durch eine entfernbare Schutzgruppe geschützt sein kann ; $R^8$ wie oben definiert ist, $A^1$ Wasserstoff oder Alkyl mit 1 bis 3 C-Atomen darstellt und $A^2$ eine derartige Gruppe ist, daß $A^1A^2CH—$ gleich der Formel (XX)

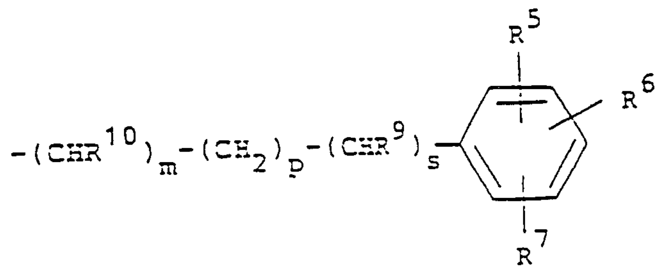

(XX)

(worin $R^5$, $R^6$, $R^7$, $R^9$, $R^{10}$, m, p und s wie oben definiert sind) ist] oder

(2) Sulfonylierung eines Amins der Formel (III)

$$H\overset{*}{N}R—Y^1—NR^8—CHA^1A^2 \qquad (III)$$

(worin $A^1$, $A^2$, R, $R^8$ und Y wie oben definiert sind) zum Einführen einer Sulfonylgruppe der Formel $Ar—SO_2—$ (worin Ar wie oben definiert ist) an dem mit einem Sternchen bezeichneten Stickstoffatom oder

(3) Alkylierung eines Sulfonamids der Formel

$$Ar—SO_2—NHR \qquad (IV)$$

(worin Ar und R wie oben definiert sind) zum Einführen einer substituierten Alkylgruppe der Formel (V)

$$—Y^1—NR^8—CHA^1A^2 \qquad (V)$$

(worin $A^1$, $A^2$, $R^8$ und $Y^1$ wie oben definiert sind) oder

(4) Alkylierung eines Amins der Formel (VI)

$$Ar—Z—Y^1—\overset{*}{N}HR^8 \qquad (VI)$$

(worin Ar, $R^8$, $Y^1$ und Z wie oben definiert sind) zum Einführen einer substituierten Alkylgruppe der Formel $—CHA^1A^2$ (worin $A^1$ und $A^2$ wie oben definiert sind) an dem mit einem Sternchen bezeichneten Stickstoffatom oder

(5) reduktive Aminierung einer Carbonylverbindung der Formel

$$A^1A^2C{=}O \qquad (VII)$$

(worin $A^1$ und $A^2$ wie oben definiert sind) mit einem Amin der Formel (VI), wie oben erläutert, oder

(6) Reduktion einer Schiff'schen Base der Formel (VIII) oder (IX)

$$Ar—Z—Y^1—N{=}CA^1A^2 \qquad (VIII)$$

$$Ar—Z—(CH_2)_q—C{=}N—CHA^1A^2 \qquad (IX)$$

(worin $A^1$, $A^2$, Ar, $Y^1$ und Z wie oben definiert sind und q 1, 2 oder 3 ist) oder eines Amids oder Thioamids der Formel (X) oder (XI)

$$Ar—Z—Y^1—NR^8—CX—A^2 \qquad (X)$$

$$Ar—Z—(CH_2)_q—CX—NR^8—CHA^1A^2 \qquad (XI)$$

(worin $A^1$, $A^2$, Ar, $R^8$, $Y^1$, Z und q wie oben definiert sind und X die Bedeutung O oder S hat) oder

(7) Alkylierung eines Amins der Formel (XII)

$$HNR^8—CHA^1A^2 \qquad (XII)$$

(worin $A^1$, $A^2$ und $R^8$ wie oben definiert sind) zum Einführen einer substituierten Alkylgruppe der Formel (XIII)

$$Ar—Z—Y^1— \qquad (XIII)$$

(worin Ar, $Y^1$ und Z wie oben definiert sind) oder

(8) reduktive Aminierung eines Aldehyds der Formel (XIV)

$$Ar—Z—(CH_2)_q—CHO \qquad (XIV)$$

(worin Ar, Z und q wie oben definiert sind) mit einem Amin der Formel (XII), wie oben erläutert, oder

(9) Alkylierung eines Sulfonamids der Formel (XV) oder (XVI)

$$Ar—SO_2—\overset{*}{N}H—Y^1—NR^8—CHA^1A^2 \qquad (XV)$$

$$Ar—\overset{*}{N}H—SO_2—Y^1—NR^8—CHA^1A^2 \qquad (XVI)$$

(worin $A^1$, $A^2$, Ar, $R^8$ und $Y^1$ wie oben definiert sind) zum Einführen einer Alkyl- oder substituierten Alkylgruppe $R^{11}$ (worin $R^{11}$ jede für R oben angegebene Bedeutung, ausgenommen Wasserstoff, hat) an dem mit einem Sternchen bezeichneten Stickstoffatom oder

(10) reduktive Aminierung eines Alkanons mit 1 bis 6 C-Atomen mit einem Amin der Formel (XVII)

$$Ar—Z—Y^1—NH—CHA^1A^2 \qquad (XVII)$$

(worin $A^1$, $A^2$, Ar, $Y^1$ und Z wie oben definiert sind) oder

(11) Reduktion eines Amids oder Thioamids der Formel (XVIII)

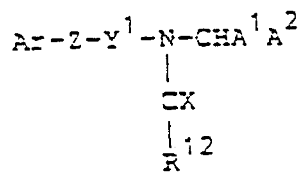

(XVIII)

(worin $A^1$, $A^2$, Ar, X, $Y^1$ und Z wie oben definiert sind und $R^{12}$ Wasserstoff oder Alkyl mit 1 bis 5 C-Atomen ist) und, wenn geeignet, Entfernung einer entfernbaren Schutzgruppe und, wenn gewünscht, Entfernung der Säure von einem Säureadditionssalz zur Bildung der freien Base, metathetisches Verdrängen zum Überführen eines Salzes in ein anderes und Neutralisation der freien Base zur Bildung eines Salzes.

2. Verfahren zur Herstellung einer Verbindung der Formel (I)

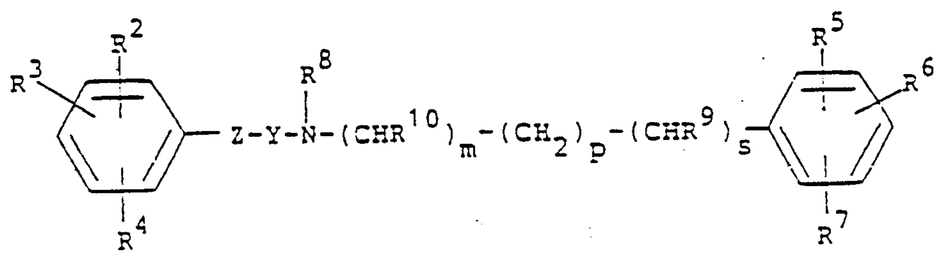

(I)

(worin Y, n, Z, R, $R^2$, $R^3$, $R^4$, $R^5$, $R^6$, $R^7$, $R^8$, $R^9$, $R^{10}$, m, p und s wie in Anspruch 1 definiert sind), welches die Entfernung einer Säure von einem Säureadditionssalz einer Verbindung der genannten Formel (I) umfaßt.

3. Verfahren zur Herstellung eines pharmazeutisch annehmbaren Salzes einer Verbindung der Formel (I)

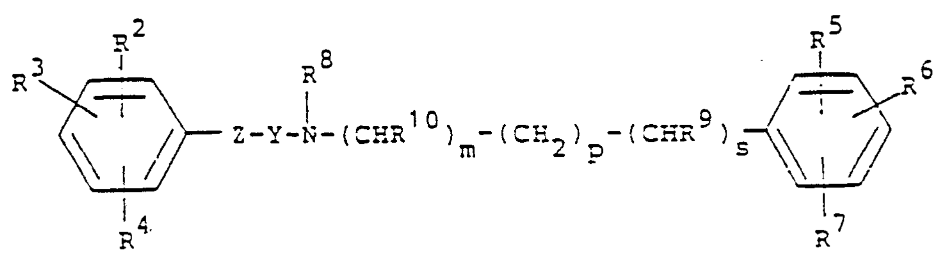

(I)

(worin Y, n, Z, R, $R^2$, $R^3$, $R^4$, $R^5$, $R^6$, $R^7$, $R^8$, $R^9$, $R^{10}$, m, p und s wie in Anspruch 1 definiert sind), welches das metathetische Verdrängen zum Überführen eines anderen Salzes der Verbindung der Formel (I) in das obgenannte pharmazeutisch annehmbare Salz oder die Neutralisation der freien Basenform der Verbindung der Formel (I) umfaßt.

4. Verfahren, wie in einem der Ansprüche 1 bis 3 beansprucht, worin die Verbindung der Formel (I) oder ein pharmazeutisch annehmbares Salz hievon zur Verwendung als Pharmazeutikum hergestellt wird.

5. Verfahren, wie in einem der Ansprüche 1 bis 4 beansprucht, worin Y die Bedeutung $-(CH_2)_n-$ hat, wobei n 2, 3 oder 4 ist ;

Z

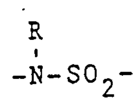

oder

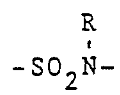

darstellt, wobei R Alkyl mit 1 bis 4 C-Atomen oder Cycloalkyl mit 3 bis 6 C-Atomen ist ; $R^8$ Alkyl mit 1 bis 3 C-Atomen darstellt ; m und s beide Null sind und p 1, 2 oder 3 ist.

6. Verfahren, wie in einem der Ansprüche 1 bis 3 beansprucht, worin eine Verbindung mit einer der Formeln (Ia), (Ib), (Ic) und (Id)

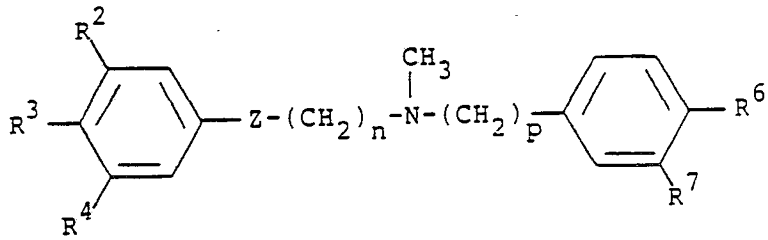

(Ia)

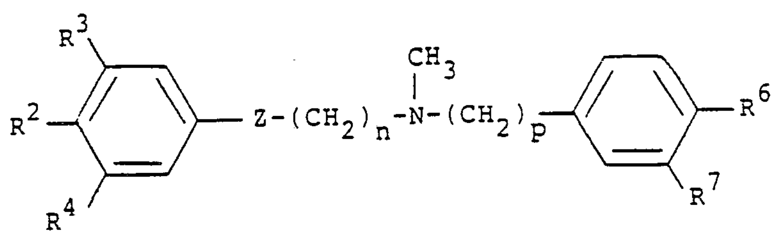

(Ib)

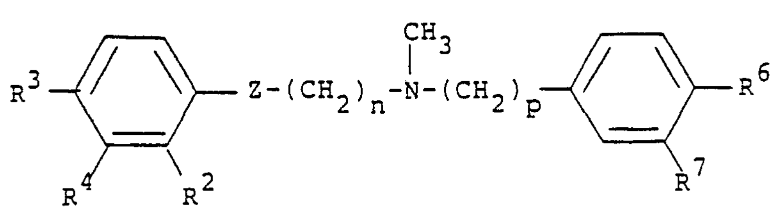

(Ic)

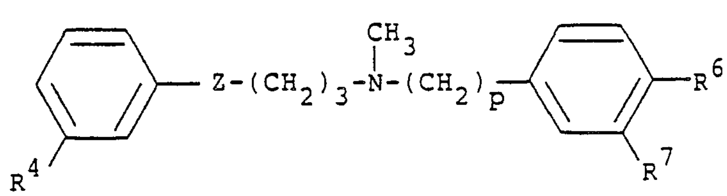

(Id)

worin

Z

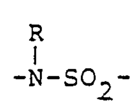

oder

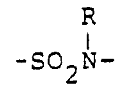

bedeutet, wobei R Alkyl mit 1 bis 4 C-Atomen ist ;

$R^2$ Wasserstoff oder Alkoxy mit 1 bis 3 C-Atomen darstellt ;

$R^3$, $R^4$, $R^6$ und $R^7$ unabhängig voneinander Alkoxy mit 1 bis 3 C-Atomen, Trifluormethyl, —Cl, —Br oder —F bedeuten ;

n 2, 3 oder 4 ist und p 1 oder 2 ist,

oder ein pharmazeutisch annehmbares Salz hievon hergestellt wird.

7. Verfahren, wie in einem der Ansprüche 1 bis 3 beansprucht, worin eine Verbindung ausgewählt aus 3,4-Dichlor-N-[4-[[2-(3,4-dimethoxyphenyl)-äthyl]-methylamino]-butyl]-N-(1-methyläthyl)-benzosulfonamid ; N-[3-[[2-(3,4-Dimethoxyphenyl)-äthyl]-methylamino]-propyl]-N-(1-methyläthyl)-2,3,4-trimethoxybenzolsulfonamid ; 3-[[2-(3,4-Dimethoxyphenyl)-äthyl]-methylamino]-N-(1-methyläthyl)-N-(3,4,5-trimethoxyphenyl)-1-propansulfonamid ; 3,4-Dichlor-N-[3-[[2-(3,4-dimethoxyphenyl)-äthyl]-methylamino]-propyl]-N-(1-methyläthyl)-benzolsulfonamid und deren pharmazeutisch annehmbaren Salzen hergestellt wird.

8. Verfahren, wie in einem der Ansprüche 1 bis 3 beansprucht, worin eine Verbindung ausgewählt aus N-(3,5-Dichlorphenyl)-3-[[2-(3,4-dimethoxyphenyl)-äthyl]-methylamino]-N-(1-methyläthyl)-1-propansulfonamid ; N-(3-Trifluormethylphenyl)-3-[[2-(3,4-dimethoxyphenyl)äthyl]-methylamino]-1-propansulfonamid ; N-(3-Trifluormethylphenyl)-3-[[2-(3,4-dimethoxyphenyl)-äthyl]-methylamino]-N-(1-methyläthyl)-1-propansulfonamid ; N-[3-[[2-(3,4-Dimethoxyphenyl)-äthyl]-methylamino]-propyl]-N-(1-methyläthyl)-3-(trifluormethyl)-benzolsulfonamid ; N-(3,4-Dichlorphenyl)-3-[[2-(3,4-dimethoxyphenyl)-äthyl]-methylamino]-N-(1-methyläthyl)-1-propansulfonamid ; N-(3,4,5-Trimethoxyphenyl)-3-[[1-(3,4,5-trimethoxyphenyl)-äthyl]-amino]-1-propansulfonamid ; N-(3,5-Dimethoxyphenyl)-3-[[2-(3,4-dimethoxyphenyl)-äthyl]-methylamino]-N-(1-methyläthyl)-1-propansulfonamid ; N-(3,4-Dimethoxyphenyl)-3-[[2-(3,4-dimethoxyphenyl)-äthyl]-methylamino]-N-(1-methyläthyl)-1-propansulfonamid ; 3-[[(3,4-Dimethoxyphenyl)-methyl]-methylamino]-N-[3-(trifluormethyl)-phenyl]-1-propansulfonamid ; N-[2-[[2-(3,4-Dimethoxyphenyl)-äthyl]-methylamino]-äthyl]-N-(1-methyläthyl)-3,4-dichlorbenzolsulfonamid ; N-(3,5-Dimethoxyphenyl)-3-[[2-(3,4-dimethoxyphenyl)-äthyl]-methylamino]-1-propansulfonamid ; N-(3,4-Dimethoxyphenyl)-3-[[2-(3,4-dimethoxyphenyl)-äthyl]-methylamino]-1-propansulfonamid ; 3-[[2-(3,4-Dimethoxyphenyl)-äthyl]-methylamino]-N-(3,4,5-trimethoxyphenyl)-1-propansulfonamid ; N-(3,5-Dichlorphenyl)-3-[[2-(3,4-dimethoxyphenyl)-äthyl]-methylamino]-1-propansulfonamid ; N-(3,4-Dichlorphenyl)-3-[[2-(3,4-dimethoxyphenyl)-äthyl]-methylamino]-1-propansulfonamid ; N-[2-[[2-(3,4-Dimethoxyphenyl)-äthyl]-methylamino]-äthyl]-3,4-dichlorbenzolsulfonamid und den pharmazeutisch annehmbaren Salzen hievon hergestellt wird.

9. Verfahren zur Herstellung einer pharmazeutischen Zusammensetzung, worin eine Verbindung der Formel (I), wie in Anspruch 1 erläutert und definiert, oder ein pharmazeutisch annehmbares Salz hievon mit einem pharmazeutisch annehmbaren Träger vereinigt oder kombiniert wird.

10. Verwendung einer Verbindung der Formel (I), wie in Anspruch 1 erläutert und definiert, oder eines pharmazeutisch annehmbaren Salzes hievon zur Herstellung eines Medikaments für die Verwendung als antiarrhythmisches Mittel.

**Revendications** (pour les Etats contractants : BE, CH, DE, FR, GB, IT, LI, LU, NL, SE)

1. Composé répondant à la formule :

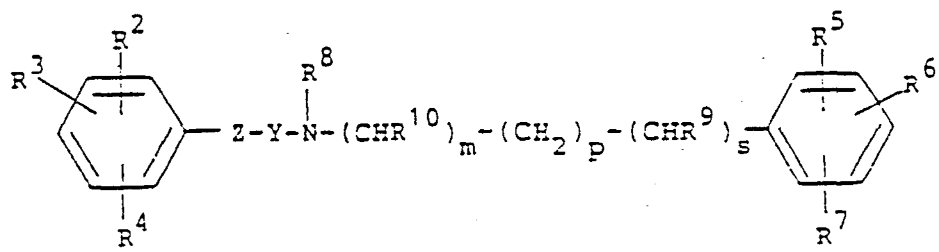

dans laquelle

Y représente un groupe $—(CH_2)_n—$ où n est égal à 1, 2, 3 ou 4, ou

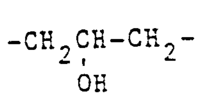

Z est le groupe

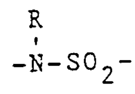

ou

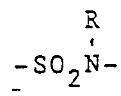

dans lequel R représente l'hydrogène, un groupe alkyle ayant 1 à 6 atomes de carbone, alkyle polyfluoré ayant 1 à 6 atomes de carbone, —CN, cyanalkyle dans lequel la portion alkyle contient 1 à 3 atomes de carbone, cycloalkyle ayant 3 à 6 atomes de carbone ou cycloalkylalkyle ayant 4 à 8 atomes de carbone ;

$R^2$, $R^3$, $R^4$, $R^5$, $R^6$ et $R^7$ représentent, indépendamment, l'hydrogène, un groupe alkoxy ayant 1 à 3 atomes de carbone, trifluorométhyle, —Cl, —Br ou —F ;

$R^8$ représente l'hydrogène ou un groupe alkyle ayant 1 à 6 atomes de carbone ;

$R^9$ représente un groupe alkyle ayant 1 à 3 atomes de carbone ;

$R^{10}$ représente un groupe alkyle ayant 1 à 3 atomes de carbone ;

m a la valeur 0 ou 1 ;

p est égal à 0, 1, 2 ou 3 ; et

s a la valeur 0 ou 1 ;

ou un de ses sels pharmaceutiquement acceptables, sous réserve que la somme de m, p et s soit égale à 1, 2, 3 ou 4 ; et que le nombre d'atomes linéaires dans le pont reliant les deux noyaux aromatiques ne soit pas supérieur à 9, et que lorsque n a la valeur 1 ou 2, deux des substituants $R^2$, $R^3$ et $R^4$ soient autres que l'hydrogène.

2. Composé répondant à la formule :

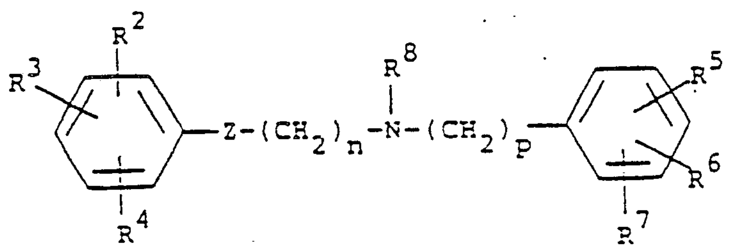

(Ie)

dans laquelle

Z représente un groupe

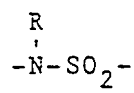

ou

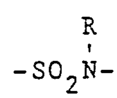

dans lequel R est un groupe alkyle ayant 1 à 4 atomes de carbone ou cycloalkyle ayant 3 à 6 atomes de carbone ;

$R^2$, $R^3$ et $R^4$ représentent, indépendamment, l'hydrogène, un groupe alkoxy ayant 1 à 3 atomes de carbone, trifluorométhyle, —Cl, —Br ou —F ;

$R^5$, $R^6$ et $R^7$ représentent, indépendamment, l'hydrogène, un groupe alkoxy ayant 1 à 3 atomes de carbone, trifluorométhyle, —Cl, —Br ou —F ;

n est égal à 2, 3 ou 4, sous réserve que, lorsque n est égal à 2, deux des substituants $R^2$, $R^3$ et $R^4$ soient autres que l'hydrogène ;

p est égal à 1, 2 ou 3 ; et

$R^8$ est un groupe alkyle ayant 1 à 3 atomes de carbone ;

ou un de ses sels pharmaceutiquement acceptables.

3. Composé répondant à la formule :

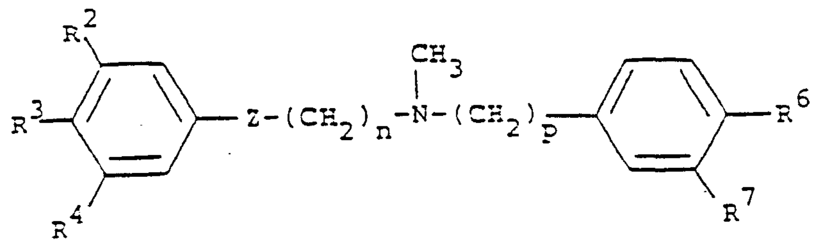 (Ia)

dans laquelle

Z représente un groupe

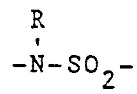

ou

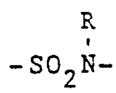

dans lequel R est un groupe alkyle ayant 1 à 4 atomes de carbone ;

$R^2$ représente l'hydrogène ou un groupe alkoxy ayant 1 à 3 atomes de carbone ;

$R^3$, $R^4$, $R^6$ et $R^7$ représentent, indépendamment, un groupe alkoxy ayant 1 à 3 atomes de carbone, trifluorométhyle, —Cl, —Br ou —F ;

n est égal à 2, 3 ou 4 ; et

p a la valeur 1 ou 2 ;

ou un de ses sels pharmaceutiquement acceptables.

4. Composé répondant à la formule :

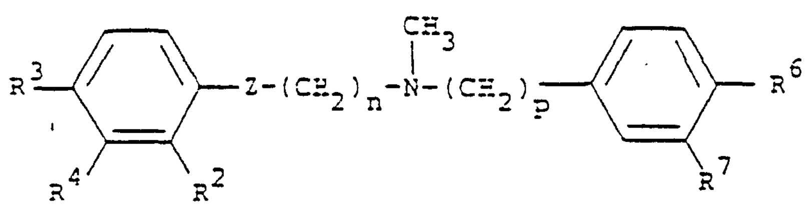 (Ic)

dans laquelle

Z représente un groupe

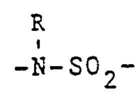

ou

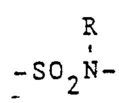

dans lequel R est un groupe alkyle ayant 1 à 4 atomes de carbone ;

$R^2$ représente l'hydrogène ou un groupe alkoxy ayant 1 à 3 atomes de carbone ;

$R^3$, $R^4$, $R^6$ et $R^7$ représentent, indépendamment, un groupe alkoxy ayant 1 à 3 atomes de carbone, trifluorométhyle, —Cl, —Br ou —F ;

n est égal à 2, 3 ou 4 ; et

p a la valeur 1 ou 2 ;

ou un de ses sels pharmaceutiquement acceptables.

5. Composé répondant à la formule :

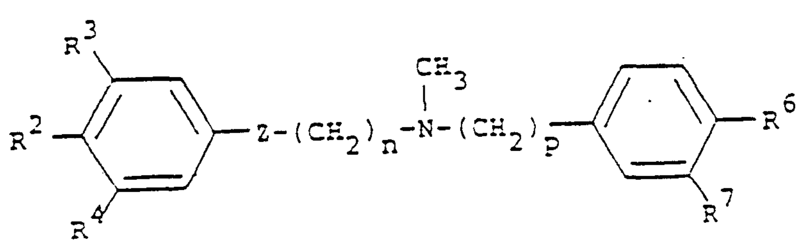 (Ib)

dans laquelle

Z représente un groupe

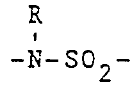

ou

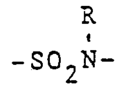

dans lequel R est un groupe alkyle ayant 1 à 4 atomes de carbone ;

$R^2$ représente l'hydrogène ou un groupe alkoxy ayant 1 à 3 atomes de carbone ;

$R^3$, $R^4$, $R^6$ et $R^7$ représentent, indépendamment, un groupe alkoxy ayant 1 à 3 atomes de carbone, trifluorométhyle, —Cl, —Br, ou —F ;

n est égal à 2, 3 ou 4 ; et

p a la valeur 1 ou 2 ;

ou un de ses sels pharmaceutiquement acceptables.

6. Composé répondant à la formule :

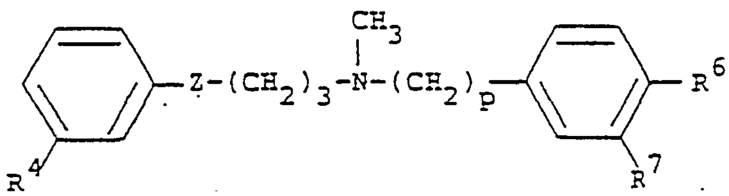

(Id)

dans laquelle

Z représente un groupe

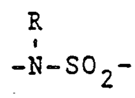

ou

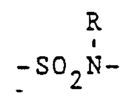

dans lequel R est un groupe alkyle ayant 1 à 4 atomes de carbone ;

$R^4$, $R^6$ et $R^7$ représentent, indépendamment, un groupe alkoxy ayant 1 à 3 atomes de carbone, trifluorométhyle, —Cl, —Br ou —F ; et

p a la valeur 1 ou 2 ;

ou un de ses sels pharmaceutiquement acceptables.

7. 3,4-dichloro-N-[4-[[2-(3,4-diméthoxyphényl)-éthyl] méthylamino] butyl]-N-(1-méthyléthyl) benzène-sulfonamide, ou un de ses sels pharmaceutiquement acceptables.

8. N-[3-[[2-(3,4-diméthoxyphényl) éthyl] méthyl-amino] propyl]-N-(1-méthyléthyl)-2,3,4-triméthoxy-benzène-sulfonamide, ou un de ses sels pharmaceutiquement acceptables.

9. 3-[[2-(3,4-diméthoxyphényl) éthyl] méthyl-amino]-N-(1-méthyléthyl)-N-(3,4,5-triméthoxyphényl)-1-propanesulfonamide, ou un de ses sels pharmaceutiquement acceptables.

10. 3-4-dichloro-N-[3-[[2-(3,4-diméthoxyphényl)-éthyl] méthylamino] propyl]-N-(1-méthyléthyl) benzène-sulfonamide, ou un de ses sels pharmaceutiquement acceptables.

11. Composé choisi entre le N-(3,5-dichlorophényl)-3-[[2-(3,4-diméthoxyphényl) éthyl] méthylamino]-N-(1-méthyléthyl)-1-propanesulfonamide ; le N-(3-trifluorométhylphényl)-3-[[2-(3,3-diméthoxyphényl) éthyl] méthyl-amino]-1-propanesulfonamide ; le N-(3-trifluorométhyl-phényl)-3-[[2-(3,4-diméthoxyphényl) éthyl] méthylamino]-N-(1-méthyléthyl)-1-propanesulfonamide ; le N-[3-[[2-(3,4-diméthoxyphényl) éthyl] méthylamino] propyl]-N-(1-méthyléthyl)-3-(trifluorométhyl) benzènesulfonamide ; le N-(3,4-dichlorophényl)-3-[[2-(3,4-diméthoxyphényl)-éthyl] méthylamino]-N-(1-méthyléthyl)-1-propanesulfonamide ; le N-(3,4,5-triméthoxyphényl)-3-[[1-(3,4,5-triméthoxyphényl) éthyl] amino]-1-propanesulfonamide ; le N-(3,5-diméthoxyphényl)-3-[[2-(3,4-diméthoxyphényl) éthyl]-méthylamino]-N-(1-méthyléthyl)-1-propanesulfonamide ; le N-(3,4-diméthoxyphényl)-3-[[2-(3,4-diméthoxyphényl)-éthyl] méthylamino]-N-(1-méthyléthyl)-1-propanesulfonamide ; le 3-[[3,4-diméthoxyphényl) méthyl] méthylamino]-N-[3-(trifluorométhyl) phényl]-1-propanesulfonamide ; le N-[2-[[2-(3,4-diméthoxyphényl) éthyl] méthylamino]-éthyl]-N-(1-méthyléthyl)-3,4-dichlorobenzènesulfonamide ; le N-(3,5-diméthoxyphényl)-3-[[2-(3,4-diméthoxyphényl)-éthyl] méthylamino]-1-propanesulfonamide ; le N-(3,4-diméthoxyphényl)-3-[[2-(3,4-diméthoxyphényl) éthyl] méthylamino]-1-propanesulfonamide ; le 3-[[2-(3,4-diméthoxyphényl)-éthyl] méthylamino]-N-(3,4,5-triméthoxyphényl)-1-propane-sulfonamide ; le N-(3,5-dichlorophényl)-3-[[2-(3,4-diméthoxyphényl) éthyl] méthylamino]-1-propanesulfonamide ; le N-(3,4-dichlorophényl)-3-[[2-(3,4-diméthoxyphényl)-éthyl] méthylamino]-1-propanesulfonamide ; le N-[2-[[2-(3,4-diméthoxyphényl) éthyl] méthylamino] éthyl]-3,4-dichlorobenzènesulfonamide, et ses sels pharmaceutiquement acceptables.

12. Composé suivant l'une quelconque des revendications 1 à 11, destiné à être utilisé comme substance pharmaceutique.

13. Composition pharmaceutique comprenant un composé suivant l'une quelconque des revendications 1 à 11, associé ou mélangé à un support pharmaceutiquement acceptable.

14. Utilisation d'un composé suivant l'une quelconque des revendications 1 à 11 pour la préparation d'un médicament destiné à être utilisé comme agent anti-arythmique.

15. Procédé de préparation d'un composé suivant la revendication 1, qui comprend

(1) la sulfonylation d'une amine aromatique répondant à la formule Ar—NHR (dans laquelle R est tel que défini dans la revendication 1 et Ar est un groupe répondant à la formule XIX)

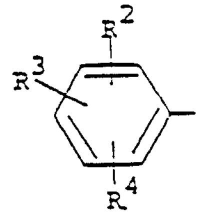

(XIX)

dans laquelle $R^2$, $R^3$ et $R^4$ sont tels que définis dans la revendication 1, pour introduire le groupe sulfonyle répondant à la formule II

$$—SO_2—Y^1—NR^8—CHA^1A^2$$

(dans laquelle $Y^1$ est identique à Y, sauf que, si un groupe hydroxy est présent dans Y, le groupe hydroxy peut être protégé par un groupe protecteur pouvant être éliminé, $R^8$ est tel que défini dans la revendication 1, $A^1$ représente l'hydrogène ou un groupe alkyle ayant 1 à 3 atomes de carbone et $A^2$ représente un groupe tel que $A^1A^2$—CH— soit identique à la formule XX

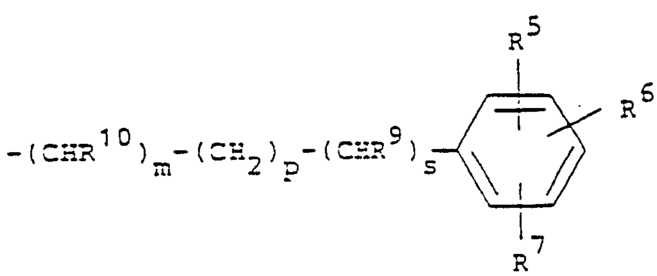

(XX)

[dans laquelle $R^5$, $R^6$, $R^7$, $R^9$, $R^{10}$, m, p et s sont tels que définis dans la revendication 1]) ; ou

(2) la sulfonylation d'une amine répondant à la formule III

$$H\overset{*}{N}R—Y^1—NR^8—CHA^1A^2 \qquad (III)$$

(dans laquelle $A^1$, $A^2$, R, $R^8$ et Y sont tels que définis ci-dessus) pour introduire un groupe sulfonyle de formule $Ar—SO_2—$ (dans laquelle Ar est tel que défini ci-dessus) au niveau de l'atome d'azote marqué avec un astérisque ; ou

(3) l'alkylation d'un sulfonamide répondant à la formule

$$Ar—SO_2—NHR \qquad (IV)$$

(dans laquelle Ar et R sont tels que définis ci-dessus) pour introduire un groupe alkyle substitué répondant à la formule V

$$-Y^1-NR^8-CHA^1A^2 \quad (V)$$

(dans laquelle $A^1$, $A^2$, $R^8$ et $Y^1$ sont tels que définis ci-dessus) ; ou

(4) l'alkylation d'une amine répondant à la formule VI

$$Ar-Z-Y^1-\overset{*}{N}HR^8 \quad (VI)$$

(dans laquelle Z est tel que défini dans la revendication 1 et Ar, $R^8$ et $Y^1$ sont tels que définis ci-dessus) pour introduire un groupe alkyle substitué de formule $-CHA^1A^2$ (dans laquelle $A^1$ et $A^2$ sont tels que définis ci-dessus) au niveau de l'atome d'azote marqué avec un astérisque ; ou

(5) l'amination réductive d'un composé carbonylé répondant à la formule

$$A^1A^2C=O \quad (VII)$$

(dans laquelle $A^1$ et $A^2$ sont tels que définis ci-dessus) avec une amine répondant à la formule VI telle qu'illustrée ci-dessus ; ou

(6) la réduction d'une base de Schiff répondant à la formule VIII ou IX

$$Ar-Z-Y^1-N=CA^1A^2 \quad (VIII)$$

$$AR-Z-(CH_2)_q-C=N-CHA^1A^2 \quad (IX)$$

(dans laquelle $A^1$, $A^2$, Ar, $Y^1$ et Z sont tels que définis ci-dessus et q est égal à 1, 2 ou 3) ou d'un amide ou thioamide répondant à la formule X ou XI

$$Ar-Z-Y^1-NR^8-CX-A^2 \quad (X)$$

$$Ar-Z-(CH_2)_q-CX-NR^8-CHA^1A^2 \quad (XI)$$

(dans laquelle $A^1$, $A^2$, Ar, $R^8$, $Y^1$, Z et q sont tels que définis ci-dessus et X représente O ou S) ; ou

(7) l'alkylation d'une amine répondant à la formule XII

$$HNR^8-CHA^1A^2 \quad (XII)$$

(dans laquelle $A^1$, $A^2$ et $R^8$ sont tels que définis ci-dessus) pour introduire un groupe alkyle substitué répondant à la formule XIII

$$Ar-Z-Y^1 \quad (XIII)$$

(dans laquelle Ar, $Y^1$ et Z sont tels que définis ci-dessus) ; ou

(8) l'amination réductive d'un aldéhyde répondant à la formule XIV

$$AR-Z-(CH_2)_q-CHO \quad (XIV)$$

(dans laquelle Ar, Z et q sont tels que définis ci-dessus) avec une amine répondant à la formule XII telle qu'illustrée ci-dessus ; ou

(9) l'alkylation d'un sulfonamide répondant à la formule XV ou XVI

$$Ar-SO_2-\overset{*}{N}H-Y^1-NR^8-CHA^1A^2 \quad (XV)$$

$$Ar-\overset{*}{N}H-SO_2-Y^1-NR^8-CHA^1A^2 \quad (XVI)$$

(dans laquelle $A^1$, $A^2$, Ar, $R^8$ et $Y^1$ sont tels que définis ci-dessus) pour introduire un groupe alkyle ou alkyle substitué $R^{11}$ ($R^{11}$ possédant l'une quelconque des significations de R donné dans la revendication 1, sauf l'hydrogène) au niveau de l'atome d'azote marqué avec un astérisque ; ou

(10) l'amination réductive d'une alcanone contenant 1 à 6 atomes de carbone avec une amine répondant à la formule XVII

$$Ar-Z-Y^1-NH-CHA^1A^2 \quad (XVII)$$

(dans laquelle $A^1$, $A^2$, Ar, $Y^1$ et Z sont tels que définis ci-dessus) ; ou

(11) la réduction d'un amide ou thioamide répondant à la formule XVIII

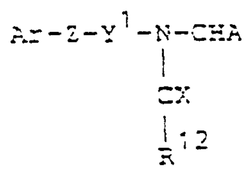

(XVIII)

(dans laquelle $A^1$, $A^2$, Ar, X, $Y^1$ et Z sont tels que définis ci-dessus et $R^{12}$ est l'hydrogène ou un groupe alkyle ayant 1 à 5 atomes de carbone) et, le cas échéant, l'élimination d'un groupe protecteur pouvant être chassé et, lorsque cela est souhaitable, l'élimination de l'acide d'un sel d'addition d'acide pour former la base libre, le déplacement métathétique pour convertir un sel en un autre sel ou la neutralisation de la base libre pour former un sel.

**Revendications** (pour l'Etat contractant AT)

1. Procédé pour la préparation d'un composé ayant la formule :

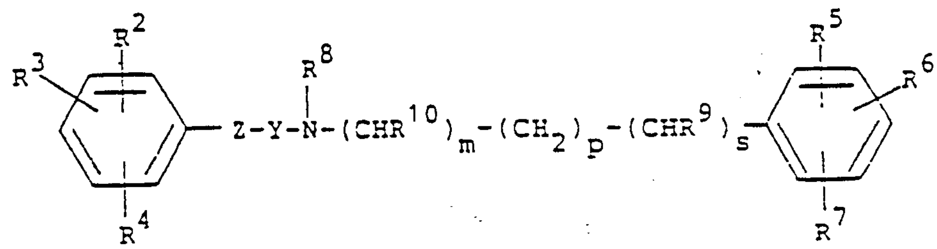

(I)

dans laquelle

Y représente un groupe $—(CH_2)_n—$ où n est égal à 1, 2, 3 ou 4, ou

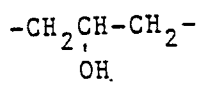

Z est le groupe

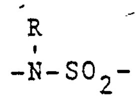

ou

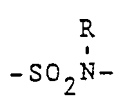

dans lequel R représente l'hydrogène, un groupe alkyle ayant 1 à 6 atomes de carbone, alkyle polyfluoré ayant 1 à 6 atomes de carbone, —CN, cyanalkyle dans lequel la portion alkyle contient 1 à 3 atomes de carbone, cycloalkyle ayant 3 à 6 atomes de carbone ou cycloalkylalkyle ayant 4 à 8 atomes de carbone ;

$R^2$, $R^3$, $R^4$, $R^5$, $R^6$ et $R^7$ représentent, indépendamment, l'hydrogène, un groupe alkoxy ayant 1 à 3 atomes de carbone, trifluorométhyle, —Cl, —Br ou —F ;

$R^8$ représente l'hydrogène ou un groupe alkyle ayant 1 à 6 atomes de carbone ;

$R^9$ représente un groupe alkyle ayant 1 à 3 atomes de carbone ;

$R^{10}$ représente un groupe alkyle ayant 1 à 3 atomes de carbone ;

m a la valeur 0 ou 1 ;

p est égal à 0, 1, 2 ou 3 ; et

s a la valeur 0 ou 1 ;

ou d'un de ses sels pharmaceutiquement acceptables, sous réserve que la somme de m, p et s soit égale à 1, 2, 3 ou 4 ; et que le nombre d'atomes linéaires dans le pont reliant les deux noyaux aromatiques ne soit pas supérieur à 9, et que lorsque n a la valeur 1 ou 2, deux des substituants $R^2$, $R^3$ et $R^4$ soient autres que l'hydrogène, qui comprend :

(1) la sulfonylation d'une amine aromatique répondant à la formule Ar—NHR (dans laquelle R est tel que défini ci-dessus et Ar est un groupe répondant à la formule XIX)

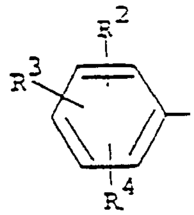

(XIX)

dans laquelle $R^2$, $R^3$ et $R^4$ sont tels que définis ci-dessus, pour introduire le groupe sulfonyle répondant à la formule II

$$—SO_2—Y^1—NR^8—CHA^1A^2$$

(dans laquelle $Y^1$ est identique à Y, sauf que, si un groupe hydroxy est présent dans Y, le groupe hydroxy peut être protégé par un groupe protecteur pouvant être éliminé, $R^8$ est tel que défini ci-dessus, $A^1$ représente l'hydrogène ou un groupe alkyle ayant 1 à 3 atomes de carbone, et $A^2$ représente un groupe tel que $A^1A^2$—CH— soit identique à la formule XX

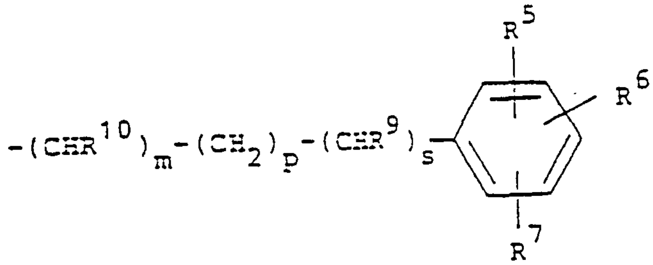

(XX)

[dans laquelle $R^5$, R, $R^7$, $R^9$, $R^{10}$, m, p et s sont tels que définis ci-dessus]) ; ou

(2) la sulfonylation d'une amine répondant à la formule III

$$H\overset{*}{N}R—Y^1—NR^8—CHA^1A^2 \qquad \text{(III)}$$

(dans laquelle $A^1$, $A^2$, R, $R^8$, et Y sont tels que définis ci-dessus) pour introduire un groupe sulfonyle de formule Ar—$SO_2$— (dans laquelle Ar est tel que défini ci-dessus) au niveau de l'atome d'azote marqué avec un astérisque ; ou

(3) l'alkylation d'un sulfonamide répondant à la formule

$$Ar—SO_2—NHR \qquad \text{(IV)}$$

(dans laquelle Ar et R sont tels que définis ci-dessus) pour introduire un groupe alkyle substitué répondant à la formule V

$$—Y^1—NR^8—CHA^1A^2 \qquad \text{(V)}$$

(dans laquelle $A^1$, $A^2$, $R^8$ et $Y^1$ sont tels que définis ci-dessus ; ou

(4) l'alkylation d'une amine répondant à la formule VI

$$Ar—Z—Y^1—\overset{*}{N}HR^8 \qquad \text{(VI)}$$

(dans laquelle Ar, $R^8$, $Y^1$ et Z sont tels que définis ci-dessus) pour introduire un groupe alkyle substitué de formule —$CHA^1A^2$ (dans laquelle $A^1$ et $A^2$ sont tels que définis ci-dessus) au niveau de l'atome d'azote marqué avec un astérisque ; ou

(5) l'amination réductive d'un composé carbonylé répondant à la formule

$$A^1A^2C{=}O \qquad \text{(VII)}$$

(dans laquelle $A^1$ et $A^2$ sont tels que définis ci-dessus) avec une amine répondant à la formule VI telle qu'illustrée ci-dessus ; ou

(6) la réduction d'une base de Schiff répondant à la formule VIII ou IX

$$Ar—Z—Y^{1}—N{=}CA^{1}A^{2} \qquad (VIII)$$

$$Ar—Z—(CH_{2})_{q}—C{=}N—CHA^{1}A^{2} \qquad (IX)$$

(dans laquelle $A^1$, $A^2$, Ar, $Y^1$ et Z sont tels que définis ci-dessus et q est égal à 1, 2 ou 3) ou d'un amide ou thioamide répondant à la formule X ou XI

$$Ar—Z—Y^{1}—NR^{8}—CX—A^{2} \qquad (X)$$

$$Ar—Z—(CH_{2})_{q}—CX—NR^{8}—CHA^{1}A^{2} \qquad (XI)$$

(dans laquelle $A^1$, $A^2$, Ar, $R^8$, $Y^1$, Z et q sont tels que définis ci-dessus et X représente O ou S) ; ou

(7) l'alkylation d'une amine répondant à la formule XII

$$HNR^{8}—CHA^{1}A^{2} \qquad (XII)$$

(dans laquelle $A^1$, $A^2$ et $R^8$ sont tels que définis ci-dessus) pour introduire un groupe alkyle substitué répondant à la formule XIII

$$Ar—Z—Y^{1}— \qquad (XIII)$$

(dans laquelle Ar, $Y^1$ et Z sont tels que définis ci-dessus) ; ou

(8) l'amination réductive d'un aldéhyde répondant à la formule XIV

$$Ar—Z—(CH_{2})_{q}—CHO \qquad (XIV)$$

(dans laquelle Ar, Z et q sont tels que définis ci-dessus) avec une amine répondant à la formule XII telle qu'illustrée ci-dessus ; ou

(9) l'alkylation d'un sulfonamide répondant à la formule XV ou XVI

$$Ar—SO_{2}—\overset{*}{N}H—Y^{1}—NR^{8}—CHA^{1}A^{2} \qquad (XV)$$

$$Ar—\overset{*}{N}H—SO_{2}—Y^{1}—NR^{8}—CHA^{1}A^{2} \qquad (XVI)$$

(dans laquelle $A^1$, $A^2$, Ar, $R^8$ et $Y^1$ sont tels que définis ci-dessus) pour introduire un groupe alkyle ou alkyle substitué $R^{11}$ ($R^{11}$ possédant l'une quelconque des significations de R données ci-dessus, sauf l'hydrogène) au niveau de l'atome d'azote marqué avec un astérisque ; ou

(10) l'amination réductive d'une alcanone contenant 1 à 6 atomes de carbone avec une amine répondant à la formule XVII

$$Ar—Z—Y^{1}—NH—CHA^{1}A^{2} \qquad (XVII)$$

(dans laquelle $A^1$, $A^2$, Ar, $Y^1$ et Z sont tels que définis ci-dessus) ; ou

(11) la réduction d'un amide ou thioamide répondant à la formule XVIII

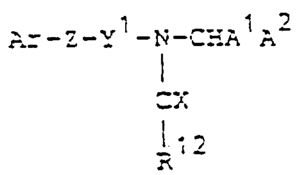

(XVIII)

(dans laquelle $A^1$, $A^2$, Ar, X, $Y^1$ et Z sont tels que définis ci-dessus et $R^{12}$ est l'hydrogène ou un groupe alkyle ayant 1 à 5 atomes de carbone) et, le cas échéant, l'élimination d'un groupe protecteur pouvant être chassé et, lorsque cela est souhaitable, l'élimination de l'acide d'un sel d'addition d'acide pour former la base libre, le déplacement métathétique pour convertir un sel en un autre sel ou la neutralisation de la base libre pour former un sel.

2. Procédé de préparation d'un composé répondant à la formule I

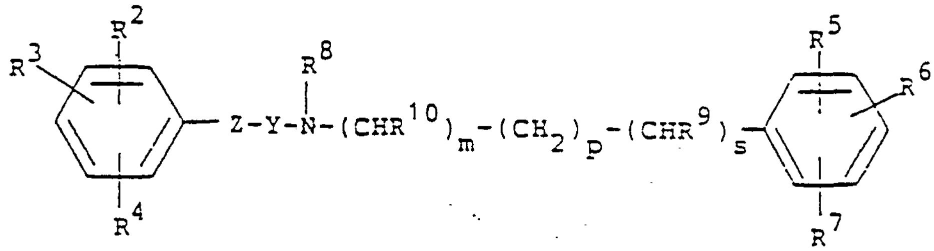

(I)

(dans laquelle, Y, n, Z, R, $R^2$, $R^3$, $R^4$, $R^5$, $R^6$, $R^7$, $R^8$, $R^9$, $R^{10}$, m, p et s sont tels que définis dans la revendication 1) qui comprend l'élimination d'un acide d'un sel d'addition d'acide d'un composé répondant à ladite formule I.

3. Procédé de préparation d'un sel pharmaceutiquement acceptable d'un composé répondant à la formule I

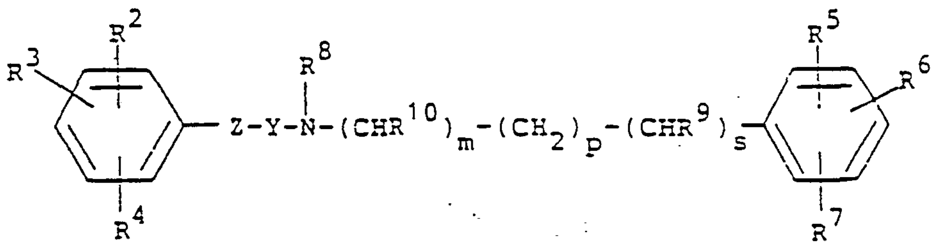

(I)

(dans laquelle Y, n, Z, R, $R^2$, $R^3$, $R^4$, $R^5$, $R^6$, $R^7$, $R^8$, $R^9$, $R^{10}$, m, p et s sont tels que définis dans la revendication 1), qui comprend un déplacement métathétique pour convertir un autre sel du composé répondant à la formule I en le sel pharmaceutiquement acceptable mentionné ci-dessus, ou une neutralisation du composé de formule I sous forme de base libre.

4. Procédé suivant l'une quelconque des revendications 1 à 3, dans lequel le composé répondant à la formule I ou un de ses sels pharmaceutiquement acceptables est préparé en vue d'une utilisation comme substance pharmaceutique.

5. Procédé suivant l'une quelconque des revendications 1 à 4, dans lequel Y représente un groupe $-(CH_2)_n$ où n est égal à 2, 3 ou 4 ; Z représente un groupe

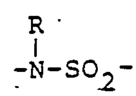

ou

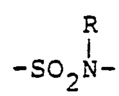

dans lequel R est un groupe alkyle ayant 1 à 4 atomes de carbone ou cycloalkyle ayant 3 à 6 atomes de carbone ; $R^8$ est un groupe alkyle ayant 1 à 3 atomes de carbone ; m et s sont tous deux égaux à 0 et p est égal à 1, 2 ou 3.

6. Procédé suivant l'une quelconque des revendications 1 à 3, dans lequel un composé répondant à l'une des formules Ia, Ib, Ic et Id

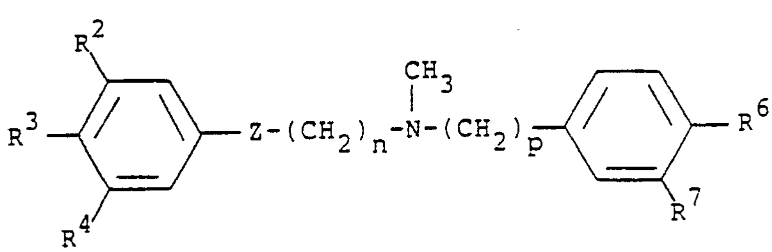

(Ia)

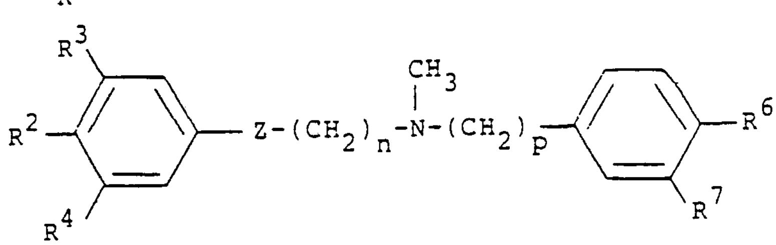

(Ib)

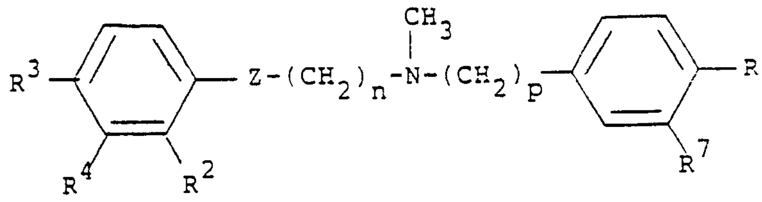

(Ic)

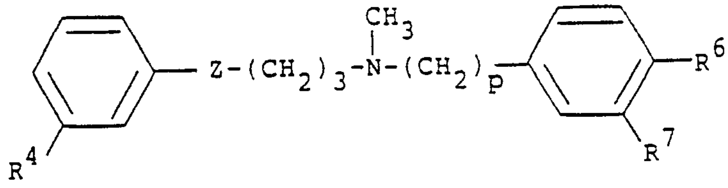

(Id)

dans lesquelles

Z représente un groupe

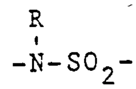

ou

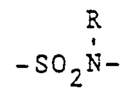

dans lequel R est un groupe alkyle ayant 1 à 4 atomes de carbone ;

$R^2$ représente l'hydrogène ou un groupe alkoxy ayant 1 à 3 atomes de carbone ;

$R^3$, $R^4$, $R^6$ et $R^7$ représentent, indépendamment, un groupe alkoxy ayant 1 à 3 atomes de carbone, trifluorométhyle, —Cl, —Br ou —F ;

n est égal à 2, 3 ou 4 et p est égal à 1 ou 2 ;

ou un de ses sels pharmaceutiquement acceptables, est préparé.

7. Procédé suivant l'une quelconque des revendications 1 à 3, dans lequel un composé choisi entre le 3,4-dichloro-N-[4-[[2-(3,4-diméthoxyphényl)-éthyl] méthylamino] butyl]-N-(1-méthyléthyl) benzènesulfonamide ; le N-[3-[[2-(3,4-diméthoxyphényl) éthyl] méthylamino]-propyl]-N-(1-méthyléthyl)-2,3,4-triméthoxybenzènesulfonamide ; le 3-[[2-(3,4-diméthoxyphényl) éthyl] méthylamino]-N-(1-méthyléthyl)-N-(3,4,5-triméthoxyphényl)-1-propane-sulfonamide ; le 3,4-dichloro-N-[3-[[2-(3,4-diméthoxyphényl) éthyl] méthylamino] propyl]-N-(1-méthyléthyl)-benzènesulfonamide, et leurs sels pharmaceutiquement acceptables, est préparé.

8. Procédé suivant l'une quelconque des revendications 1 à 3, dans lequel un composé choisi entre le N-(3,5-dichlorophényl)-3-[[2-(3,4-diméthoxyphényl) éthyl] méthylamino]-N-(1-méthyléthyl)-1-propanesulfonamide ; le N-(3-trifluorométhylphényl)-3-[[2-(3,4-diméthoxyphényl) éthyl] méthylamino]-1-propanesulfonamide ; le N-(3-trifluorométhylphényl)-3-[[2-(3,4-diméthoxyphényl) éthyl] méthylamino]-N-(1-méthyléthyl)-1-propanesulfonamide ; le N-[3-[[2-(3,4-diméthoxyphényl)-éthyl] méthylamino] propyl]-N-(1-méthyléthyl)-3-(trifluorométhyl) benzènesulfonamide ; le N-(3,4-dichlorophényl)-3-[[2-(3,4-diméthoxyphényl) éthyl] méthylamino]-N-(1-méthyléthyl)-1-propanesulfonamide ; le N-(3,4,5-triméthoxyphényl)-3-[[1-(3,4,5-triméthoxyphényl) éthyl] amino]-1-propanesulfonamide ; le N-(3,5-diméthoxyphényl)-3-[[2-(3,4-diméthoxyphényl) éthyl] méthylamino]-N-(1-méthyléthyl)-1-propanesulfonamide ; le N-(3,4-diméthoxyphényl)-3-[[2-(3,4-diméthoxyphényl) éthyl] méthylamino]-N-(1-méthyléthyl)-1-propanesulfonamide ; le 3-[[(3,4-diméthoxyphényl) méthyl] méthylamino]-N-(3-(trifluoro-méthyl) phényl]-1-propanesulfonamide ; le N-[2-[[2-(3,4-diméthoxyphényl) éthyl] méthylamino] éthyl]-N-(1-méthyléthyl)-3,4-dichlorobenzènesulfonamide ; le N-(3,5-diméthoxyphényl)-3-[[2-(3,4-diméthoxyphényl) éthyl]-méthylamino]-1-propanesulfonamide ; le N-(3,4-diméthoxyphényl)-3-[[2-(3,4-diméthoxyphényl) éthyl] méthylamino]-1-propanesulfonamide ; le 3-[[2-(3,4-diméthoxyphényl)-éthyl] méthyalmino]-N-(3,4,5-triméthoxyphényl)-1-propane-sulfonamide ; le N-(3,5-dichlorophényl)-3-[[2-(3,4-diméthoxyphényl) éthyl] méthylamino]-1-propanesulfonamide ; le N-(3,4-dichlorophényl)-3-[[2-(3,4-diméthoxyphényl)-éthyl] méthylamino]-1-propanesulfonamide ; le N-[2-[[-(3,4-diméthoxyphényl) éthyl] méthylamino] éthyl]-3,4-dichlorobenzènesulfonamide, et leurs sels pharmaceutiquement acceptables, est préparé.

9. Procédé de préparation d'une composition pharmaceutique dans laquelle un composé répondant à la formule I telle qu'illustrée et définie dans la revendication 1 ou un de ses sels pharmaceutiquement acceptables est associé ou mélangé à un support pharmaceutiquement acceptable.

10. Utilisation d'un composé répondant à la formule I telle qu'illustrée et définie dans la revendication 1 ou d'un de ses sels pharmaceutiquement acceptables pour la préparation d'un médicament destiné à être utilisé comme agent anti-arythmique.